(19) Europäisches Patentamt
European Patent Office
Office européen des brevets

(11) EP 0 900 380 B1

(12) EUROPEAN PATENT SPECIFICATION

(45) Date of publication and mention of the grant of the patent: **09.07.2003 Bulletin 2003/28**

(21) Application number: **97919749.8**

(22) Date of filing: **28.04.1997**

(51) Int Cl.[7]: **G01N 33/569**, G01N 33/557, A61K 39/00, C07K 14/00, C12N 15/00, C12N 15/83

(86) International application number: **PCT/NL97/00229**

(87) International publication number: **WO 97/041440 (06.11.1997 Gazette 1997/47)**

# (54) METHODS FOR SELECTING AND PRODUCING T CELL PEPTIDE EPITOPES AND VACCINES INCORPORATING SAID SELECTED EPITOPES

VERFAHREN ZUR SELEKTION UND PRODUKTION VON T-ZELL-PEPTIDE EPITOPE UND VAKZINE MIT DIESE EPITOPE

PROCEDES DE SELECTION ET DE PRODUCTION D'EPITOPES PEPTIDIQUES DE LYMPHOCYTES T ET VACCINS CONTENANT LESDITS EPITOPES SELECTIONNES

(84) Designated Contracting States:
**AT BE CH DE DK ES FI FR GB GR IE IT LI LU NL PT SE**

(30) Priority: **26.04.1996 EP 96201145**
**23.12.1996 EP 96203670**

(43) Date of publication of application:
**10.03.1999 Bulletin 1999/10**

(73) Proprietors:
- **Rijksuniversiteit te Leiden 2312 AV Leiden (NL)**
- **SEED CAPITAL INVESTMENTS( SCI) B.V. 3527 GA Utrecht (NL)**

(72) Inventors:
- **VAN DER BURG, Sjoerd, Henricus NL-2742 BT Waddinxveen (NL)**
- **KAST, Wybe, Martin Willowbrook, IL 60150 (US)**
- **TOES, Reinaldus, Everardus, Maria NL-1057 WN Amsterdam (NL)**
- **OFFRINGA, Rienk NL-2313 SJ Leiden (NL)**
- **MELIEF, Cornelius, Johannes, Maria NL-2012 KC Haarlem (NL)**

(74) Representative: **Prins, Adrianus Willem et al Vereenigde, Nieuwe Parklaan 97 2587 BN Den Haag (NL)**

(56) References cited:
**WO-A-94/21287** **WO-A-94/26785**
**WO-A-95/09642** **WO-A-96/22067**

- **NATURE (LONDON) (1994), 371(6494), 250-2 CODEN: NATUAS;ISSN: 0028-0836, XP000604820 NELSON, CHRISTOPHER A. ET AL: "Peptides determine the lifespan of MHC class II molecules in the antigen-presenting cell"**
- **THE JOURNAL OF IMMUNOLOGY, vol. 156, no. 9, 1 May 1996, pages 1308-1314, XP002040034 VAN DER BURG, S.H., ET AL.: "Immunogenecity of peptides bound to MHC Class I Molecules depends on the MHC-peptide Complex stability"**
- **THE JOURNAL OF IMMUNOLOGY, vol. 157, 1996, pages 822-826, XP002039433 THOMSON, S.A. ET AL.: "Recombinant polyepitope vaccines for the delivery of multiple CD8 cytotoxic T Cell epitopes."**
- **JOURNAL OF VIROLOGY, vol. 67, no. 1, 1 January 1993, pages 348-352, XP000674226 WHITTON J L ET AL: "A "STRING-OF-BEADS" VACCINE, COMPRISING LINKED MINIGENES, CONFERS PROTECTION FROM LETHAL-DOSE VIRUS CHALLENGE"**
- **JOURNAL OF IMMUNOLOGY, vol. 149, no. 11, 1 December 1992, pages 3580-3587, XP000371892 PARKER K C ET AL: "SEQUENCE MOTIFS IMPORTANT FOR PEPTIDE BINDING TO THE HUMAN MHC CLASS I MOLECULE, HLA-A2"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 900 380 B1

- IMMUNOGENETICS (1995), 42(5), 392-7 CODEN: IMNGBK;ISSN: 0093-7711, XP000603776 DAVENPORT, MILES P. ET AL: "An empirical method for the prediction of T-cell epitopes"
- ACTA CRYSTALLOGR., SECT. D: BIOL. CRYSTALLOGR. CODEN: ABCRE6;ISSN: 0907-4449, vol. D51, no. 4, 1995, pages 541-549, XP000603826 SCAPOZZA, LEONARDO ET AL: "Molecular dynamics and structure-based drug design for predicting non-natural nonapeptide binding to a class I MHC protein"
- MOLECULAR IMMUNOLOGY, vol. 31, no. 11, 1994, pages 813-822, XP000603774 A. SETTE ET AL.,: "peptide binding to the most frequent HL-A Class I alleles measured by quantitative molecular binding assays"
- JOURNAL OF IMMUNOTHERAPY, vol. 14, no. 2, 1993, pages 121-126, XP002015226 NIJMAN, H.W. ET AL.: "Characterization of Cytotoxic T lymphocyte epitopes of a self-protein, p53 and a non-self-protein, influenzy matrix: relationship between major histocompatibility complex peptide binding affinity and immune responsiveness to peptides."
- HUMAN IMMUNOLOGY, vol. 43, no. 1, May 1995, pages 13-18, XP000603786 D'AMARO,J. ET AL.: "A computer Program for predicting possible Cytotoxic T lymphocyte epitopes based on HLA Class I Peptide-binding motifs"

## Description

### §1 Field of the invention

The present invention relates to the field of molecular biology and immunology. In particular it relates to vaccines and methods for providing vaccines which elicit immune responses when administered to a mammal, in particular a human. The preferred elicited immune response is a T cell response, elicited by peptide T cell epitopes. These vaccines find their application in many fields ranging from cancer treatments to treatments or prophylaxis of infectious diseases such as Aids. The present invention provides novel methods for selecting the peptide sequences from an intact antigen which will lead to a proper (T cell) immune response upon administration in a suitable vehicle. The epitopes and vaccines are, of course, also part of the present invention.

### §2 Background of the invention

Virtually all currently available vaccines are not rationally designed in the sense of detailed knowledge of minimal essential epitopes and the rules of antigen processing and presentation. Rather, the available vaccines are based on empirical knowledge of protection. A major objective of the present invention is to develop a new generation of more rationally designed vaccines which are effective, safe, easy to manufacture and standardise, stable, inexpensive and associated with long lasting protection. This objective is achieved by employing our knowledge on the biochemistry of antigen processing and presentation in general, and in dendritic cells in particular, in the selection of peptide epitopes. Subsequently, selected peptide epitopes are incorporated into various types of vaccines and tested for efficacy in for instance HLA-transgenic mouse models.

The selection of peptide epitopes for a given combination of antigen and HLA class I molecule according to the invention may be divided in the following subsequent steps:

1. Computer prediction of peptides within the primary sequence of the antigen that are most likely to bind to the HLA class I molecule concerned, by comparison with the relevant motif for MHC class I binding (1).
2. Measurement of the actual binding of the selected peptides to the MHC molecule concerned using assays that determine HLA-peptide binding and stability of the HLA-peptide complex (2, 3) (Examples 1 and 2 herein).
3. Screening of the peptides (selected by steps 1 & 2) and their flanking sequences (in the context of the intact antigen) for compliance with the rules for proteasome cleavage of natural protein sequences (4).
4. Screening of the peptides (selected by steps 1 & 2) and their flanking sequences (in the context of the intact antigen) for compliance with the rules for effective peptide transport and loading into HLA class I molecules (5).

Selected peptide epitopes (see steps 1-4) are incorporated into the following prototype vaccines, the efficacy of which is compared in the appropriate HLA transgenic mouse model:

i. Mixture of synthetic peptides in adjuvants.
ii. Mixture of synthetic peptides loaded onto dendritic cells.
iii. ecombinant protein, synthesized in - and purified from - E.coli, consisting of a string bead Rarrangement of peptide-epitopes which are separated from each other by proteolytic cleavage sites. Protein administered in adjuvants.
iv. Recombinant protein (see iii; mannosylated) loaded onto dendritic cells.
v. Recombinant DNA construct (naked DNA) that encodes string-beads of peptide epitopes which are separated by proteolytic cleavage sites.
vi. Recombinant Canary pox virus that encodes string-beads of peptide epitopes which are separated by proteolytic cleavage sites.
vii. Recombinant human adenovirus that encodes string-beads of peptide epitopes which are separated by proteolytic cleavage sites.

Efficacy of the various vaccination protocols is assayed by restimulation in mixed lymphocyte cultures of spleen cells of the immunized animals with autologous LPS B-cell blasts that are loaded with the relevant peptide(s), followed by measurement of the reactivity of the resulting T cell cultures against target cells that either present synthetic peptides or the naturally processed epitopes.

The peptide epitopes are also used for the induction of antigen-specific T cell activity in HLA-transgenic mixed lymphocyte cultures in vitro. To that end, the peptide(s) of choice are loaded onto either syngeneic LPS B-cell blasts or dendritic cells. These cells are irradiated and used as stimulator cells with nylonwool passed spleen cells of HLA-transgenic mice. After in vitro stimulation for one or two weeks, the reactivity of the resulting T cell populations can be measured against target cells that either present synthetic peptides or the naturally processed epitopes.

Rational design of vaccines has clear advantages. Safety is one. For example DNA or viral vector vaccines for HPV16 E6 and E7 are intrinsically unsafe if such vaccines contain functional oncogenes, but safe if the DNA or viral vector encodes string beads of epitopes, a preferred embodiment of the present invention. Additional advantages are effectiveness and simplicity. Only effectively immunizing components are included. Irrelevant sequences are deleted, easing manufacture and standardization, enhancing stability and decreasing cost.

The design of effective T cell epitope vaccines hinges on the accurate selection of immunogenic peptides. By means of the current invention.(a method that analyzes the stability of peptide-MHC complexes at the surface of antigen-presenting cells) we have considerably improved the selection procedure. Moreover, we have also significantly improved the procedure by which poly- T cell-epitope-containing vaccines induce strong anti-tumor and anti-viral immune responses.

The present invention thus provides a method for the selection of T cell peptide epitopes present in polypeptide antigens comprising identification of peptides in the primary sequence of the antigen having a binding motif and size for binding to a HLA class I molecule, measuring the binding of said identified peptides to MHC class I molecules, whereby the stability of the complex of the peptide and the MHC class I molecule is measured on intact cells carrying said MHC class I molecule at their surfaces. Moreover, the present invention provides a new method for the application of identified T cell epitopes comprising incorporation of a multitude of T cell epitopes in a string-of-bead construct, in which the T cell epitopes preferably are linked to each other by a spacer-sequence that ensures efficient processing and presentation of the relevant T cell epitopes.

**§3 Summary of the invention**

Peptide-binding to MHC under physiological conditions is governed by dynamic balance between association and dissociation of the MHC-peptide complexes. Both the capacity of a peptide to bind to an MHC molecule and the stability of the resulting MHC-peptide complex over time will determine the amount of a given peptide-MHC complex at the surface of a target/stimulator cells and, thereby, the chance that this configuration will be detected by responding T lymphocytes. Several assays have been set up to measure these parameters in the context of the human and the murine immune system. These assays are especially suitable for measuring peptide binding and stability of peptide-MHC complexes in the context of various HLA class I molecules:

i. The T2-assay, that measures binding of peptides to empty MHC class I MHC molecules at the surface of the processing defective cell line 174CEM.T2 (T2) (2).

ii. An assay that measures binding of peptides to soluble class I MHC molecules that have been isolated from appropriate cell lines (6).

iii. An assay that measures binding of peptides to soluble class I MHC molecules that have been isolated as recombinant proteins from E. coli cultures that overexpress these proteins (7, 8).

iv. An HLA class I peptide-binding assay based on competition for binding to class I molecules on intact human B cells (3) (Example 1 herein).

v. An HLA class I peptide-binding assay that measures the stability of class I MHC-peptide complexes on intact human B cells (a major object of the present invention; see Examples 2 and 3 herein).

In the present invention the latter assay is provided: a novel assay that measures MHC-class I peptide complex stability on intact cells carrying said MHC claim I molecule at their surface. The binding affinity of peptides to MHC molecules under physiological conditions is a dynamic equilibrium between association and dissociation of the trimolecular complex of peptide, MHC class I heavy chain and β2-microglobulin. Currently, the affinity of peptides for a given class I molecule is based on assays that employ either cell-bound MHC class I molecules or purified "cell-free" MHC class I molecules. Affinity is measured by comparative capacity of peptides to upregulate MHC class I molecules on the surface of processing defective cells (2) or by their ability to compete with high affinity reference peptides (3, 9, 10). However, these assays hardly take into account the stability of peptide-MHC complexes under physiological conditions due to short incubation time, continuous presence of high concentrations of exogenous peptide, or reduced temperature. Recently, we have measured the fate of existing MHC-peptide complexes over time under more physiological conditions. Peptides displaying comparable binding affinities as measured in previous assays, showed marked differences with respect to the stability of the peptide-MHC complexes. Moreover, stability of the peptide-MHC complex correlated better with the immunogenicity of the peptide than binding affinity (see Examples 2 and 3 of this patent application).

Examples of self peptides displaying low binding affinity that represent immunogenic T cell epitopes are peptides derived from MART-1 (AAGIGILTV / ILTVILGVL) (11), Pmel17/gp100 (YLEPGPVTA) (12) and p53 (LLGRNSFEV) (13). These peptides were enclosed in this category based on results obtained in classical binding assays. However, measurement of the stability of the relevant peptide-MHC complexes revealed that the stability of these complexes is comparable to that of known epitopes of viral origin (see Examples 2 and 3 of this patent application). Therefore, these peptides, although somewhat sluggish in mounting the MHC molecule, should not be regarded as displaying a low affinity for the presenting MHC molecule. This notion confirms that, in addition to peptide binding affinity, stability of the peptide-MHC complex is an important parameter for the identification of immunogenic peptide-epitopes. As MHC-peptide complex stability correlates even better with the immunogenicity of the peptide than binding affinity, assays measuring complex stability represent an important and indispensable new step in the sequence of procedures that is used to identify immunogenic peptide epitopes from primary amino acid sequences.

Another important embodiment of the present invention is provided by the innovative method that induces T cell reactivity against multiple pre-selected T cell epitopes by immunization with a recombinant adenovirus (rAd) vector that contains multipe T cell epitopes in a string-of-bead fashion in which the T cell epitopes are linked to each other by proteolytic cleavage sites. The linkage of T cell epitopes by spacer sequences ensures that the T cell epitopes are efficiently processed and presented to T cell. Therefore, the incorporation of multiple T cell epitopes spaced by linker-sequences preferably into recombinant adenovectors represents an important and powerful new approach for the induction of strong anti-viral and anti-tumor T cell immunity that is directed against multiple T cell targets.

## §4 Detailed description of the invention

Herein we describe an assay that measures the stability of peptide-MHC complexes at the surface of human B cell lines. This assay, which is used to identify immunogenic peptide-epitopes, constitutes a major step forward on the road towards rational vaccine design. The novel methodology described herein is based on a binding assay that measures peptide binding on intact human B cells. This assay is, therefore, separately described in the following paragraph (§4.1). This binding assay has been published previously. The stability assay, which utilizes an innovative combination of steps is subsequently described (§4.2). The part of the patent application describing a vaccination strategy using rAd harbouring string-of-bead constructs encoding several pre-selected T cell epitopes is given in § 4.5.

### §4.1 An HLA class I peptide-binding assay based on competition for binding to class I molecules on intact human B cells.

Peptide-binding assays employ either cell-bound MHC class I molecules (2, 3) or purified "cell-free" MHC class I molecules (6). Assays relying on cell-bound MHC class I molecules are based on upregulation (2) or reconstitution of MHC class I molecules (3) as detected by MHC class I conformation-specific antibodies. Assays relying on cell-bound MHC class II molecules are also described in the art. For instance, Nelson et al (75) describe a peptide binding assay for MHC class II that makes use of intact cells in combination of radiolabeled peptides. Cell-free systems are quantitative and can make use of purified MHC molecules to which labeled reference peptides are bound in a competition set-up (6). Parker et al (76) disclose a method for comparing the binding properties of different peptides to the human MHC class I molecule HLA-A2. For this purpose a cell-free system is used, in which the class I complex is reconstituted from the class I heavy chain, peptide, and β2m. Peptide binding is detected by measuring the incorporation of $^{125}$I-labeled β2-microglobulin into the class I complex, which can be analyzed by native isoelectric focusing gels. Purification of MHC class I molecules, however, is laborious and conformational changes may occur during purification and/or storage. The peptide binding assay we use to identify peptides which bind to various HLA class I molecules utilizes fluorescein-labeled reference peptides that bind to HLA class I molecules on HLA-homozygous B-cell lines, of which the bound peptides have been removed by mild-acid treatment. The use of intact human B cells as tools in peptide binding assays has several clear advantages:

* EBV-transformed B cells can be easily grown to high numbers without the use of exclusive (=expensive) tissue culture media or growth factors.
* EBV-transformed B cells express high levels of class I MHC; no treatment with lymphokines such as IFNγ is needed to reach these high expression levels.
* the mild-acid treated B cells are easily prepared; including harvesting of the B cells from cultures, the amount of stripped B cells needed for an average assay will be ready for use within 30 minutes.
* an almost infinite repertoire of EBV-transformed human B cell lines, expressing various combinations of class I MHC molecules, is available in many laboratories throughout the world
* when necessary, new EBV-transformed B cell lines can readily be made within one month. EBV-transformation of human B cells is a very straightforward procedure that is routinely performed in many laboratories throughout the world.

We have shown that the binding of fluorescein-labeled peptides to these peptide-stripped HLA class I molecules is specific and allows the semi-quantitative determination of the binding-capacity of peptides. The kinetics of peptide-binding to these peptide-stripped HLA class I molecules is comparable to that of soluble HLA class I molecules and independent of biosynthesis of new HLA class I molecules. This assay was optimized and validated with peptides of known binding capacity to either HLA-A*0101, HLA-A*0201, HLA-A*0301, HLA-A*1101 or HLA-A*2401 (3, 6) (our additional unpublished data). Furthermore, this assay was among others applied in the identification of potential HLA-A0301-restricted conserved CTL epitopes derived from HIV-1 polymerase (3).

**Example 1.**

**Validation of a peptide-binding assay employing the HLA-A0201 and A0301 molecules on intact human B cells** (adapted from (3)).

Materials and methods

*Cell lines*

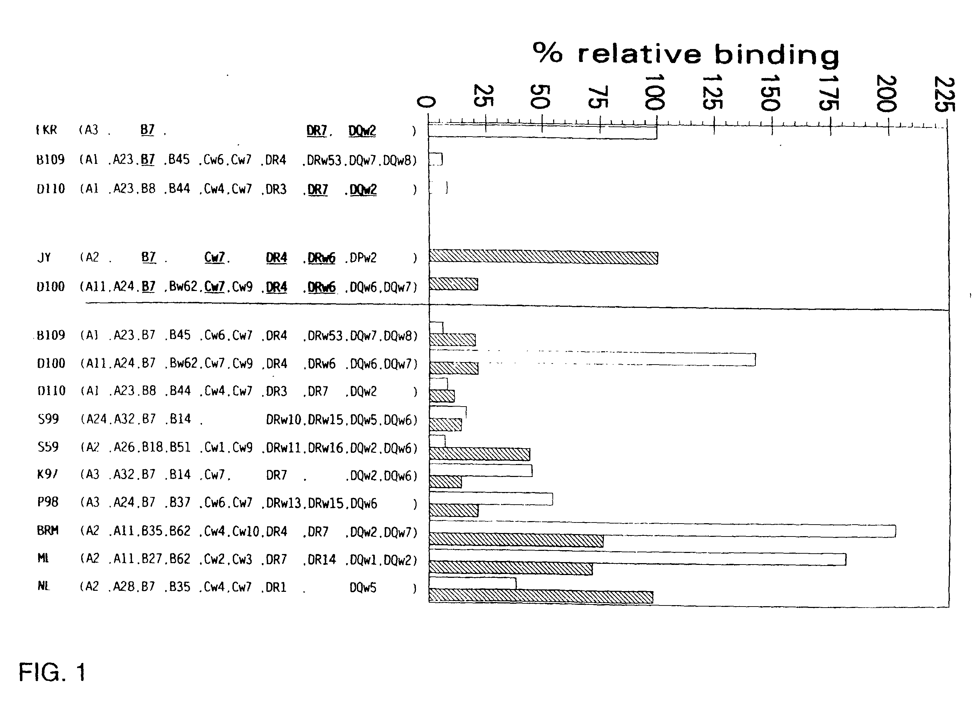

The EBV transformed B cell lines (B-LCL) used for the competition assays are *JY* (HLA type: A*0201, B7, Cw7, DR4, DRw6, DPw2) and *EKR* (HLA type: A3, B7, DR7, DQw2).
The B-LCL used to confirm specific binding of reference peptides are *B109 , BRM, D100, D110, K97, ML, NL, P98, S59* and *S99.* The HLA type of these cell lines is given in fig. 1.

*Peptides*

Fluorescein (FL)-labeled reference peptides were synthesized as Cys-derivative. Labeling was performed with 4-(iodoacetamido) fluorescein (Fluka Chemie AG, Buchs, Switzerland) at pH 7.5 (Na-phospate in water/acetonitrile 1:1). The labeled peptides were desalted over Sephadex G-10 and further purified by C18 RP-HPLC. Labeled peptides were characterized by MALDI-MS (Lasermat, Finnigan, UK). The reference peptide used for HLA-A*0301 binding was KVFPC(FL)ALINK ($MH^+$calc=1521.8, $MH^+$meas=1521.4), the reference peptide for HLA-A*0201 was FLPSDC(FL) FPSV ($MH^+$calc=1500.6, $MH^+$meas=1500.1).

The reference peptides used for binding to HLA-A*0301 or HLA-A*0201 were published by Sette et al. (14). In both peptides these investigators introduced a tyrosine which they used to tag a radioactive label to the peptide. We have substituted this tyrosine for a cysteine. The cysteine allowed the conjugation of 4-(iodoacetamido)fluorescein.

The polymerase amino-acid sequences of 14 different full length sequenced HIV-1 virus strains: LAI, MN, NL43, OYI, SF2, RF, MAL, D31, CAM1, HAN, ELI, NDK, JRCSF and JRFL (15) were screened for possible HLA-A*0301 restricted CTL epitopes using a scoring system (1). The HLA-A*0301 motif used was based on the studies of Kubo et al. (16) and Engelhard (17). At the anchor at position 2 a L, I, V or M and at the C-terminal anchor a K, R or Y was preferred. Peptides were synthesized that contained the mentioned residues at both anchor positions and were completely conserved among all 14 HIV-1 strains.

Peptides were synthesized by solid-phase strategies on an automated multiple peptide synthesizer (Abimed AMS 422, Langenfeld, Germany) using Fmoc-chemistry. Peptides were analyzed by reverse phase HPLC, dissolved in 20 μl dimethyl sulfoxide (DMSO), diluted in 0.9% NaCl to a peptide concentration of 5 mg/ml and stored at -20°C before usage.

*Mild-acid treatment of B-LCL*

Mild-acid treatment of HLA-A2 or HLA-A3 on B-LCL was performed according to Bremers modification (18) of the procedure of Storkus et al. (19). Briefly, cells were washed twice with PBS and then put to rest on ice for 5 minutes. The cells were then treated 90 seconds with ice-cold citric-acid-$Na_2HPO_4$ buffer (mixture of an equal volume of 0.263 M citric acid and 0.123 M $Na_2HPO_4$) (20). For HLA-A3 the buffer was adjusted to pH=2.9 and to pH=3.2 for HLA-A2, these pH differences are essential for optimal elution of bound peptides and reconstitution of the MHC class I molecule with the exogenous added peptide (18). Immediately thereafter the eluted cells were buffered with cold ISCOVE's modified Dulbecco's medium (IMDM), washed with IMDM and resuspended at 700.000 cells/ml in IMDM + 1,5 Ug/ml β2m (Sigma, St. Louis, USA).

*Peptide competition assay*

For competition assays, 25 μl FL-labeled reference peptide (end conc:150 nM in PBS) was incubated with 25 μl competitor peptide (different end concentrations in PBS) in a 96-well U-bottom plate (Costar, Cambridge, Massachusetts, USA). 100 µl of the mild-acid treated B-LCL (A2:JY, A3:EKR) was added to these wells.

The mixture was incubated for 3 or 24 hr at 4°C or 26°C, washed twice with PBS containing 1% BSA (PBA1%), resuspended in PBA1% containing 0.5% paraformaldehyde and analyzed at a FACscan (Becton-Dickinson, Etten-Leur, the Netherlands).

The mean-fluorescence (MF) value obtained in the experiment without competitor peptide was regarded as maximal binding and equated to 0% inhibition, the MF obtained from the experiment without reference peptide was equated to 100% inhibition.

% inhibition of binding was calculated using the following formula:

(1- (MF 150nM reference & competitor peptide - MF no

reference peptide) - (MF 150nM reference - MF no reference peptide)) x 100%

In experiments where no competitor peptide was added the fluorescence index (FI) was calculated to indicate how much fluorescence above the background (no reference peptide) was measured. The FI = (MF sample - MF background)/ MF background.

To block protein synthesis in B-LCL a final concentration of 100 µM emetine (Sigma, St Louis, USA) was used, as shown previously (20).

<u>Results</u>

*Sensitivity and specificity of FL-labeled reference peptides binding to HLA class I*

The reference peptides binding to HLA-A*0201 and HLA-A*0301 were described and used in a molecular binding assay by Sette et al. (14) In both peptides a tyrosine was used to tag a radioactive label to the peptide. We substituted this tyrosine with cysteine, to which 4-(iodoacetamido)fluorescein was conjugated.

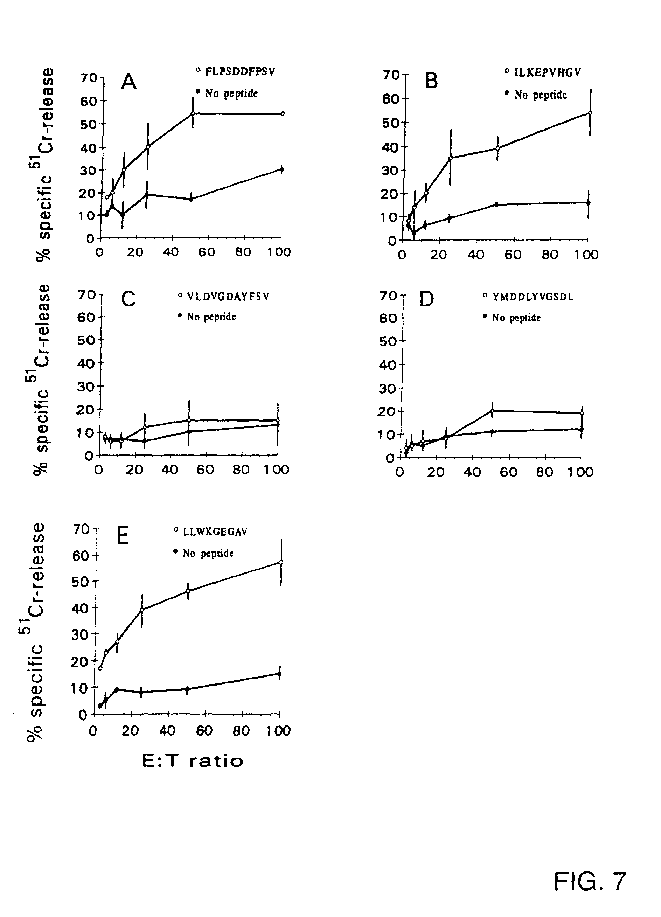

The amount of fluorescent peptide needed for the competition assay was established. For this purpose a peptide titration was performed. After incubation of three hours at 26°C the mean fluorescence (MF) was measured. At concentrations from 2 nM to 100 nM a sharp increase in MF was found for the HLA-A*0201 reference peptide and from 2 nM to 150 nM for the HLA-A*0301 reference peptide (data not shown). Mild-acid treatment of the B-cells before incubation with FL-labeled reference peptide resulted in a higher fluorescence maximum and also sharper increase of the MF at low peptide concentrations (fig. 7).

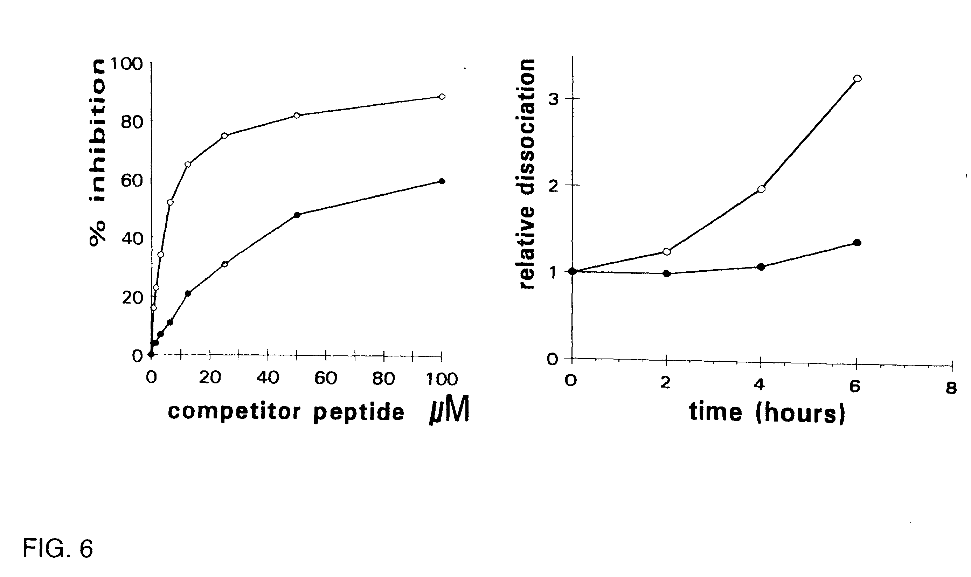

In order to investigate if aspecific peptide-binding to cell components, including other HLA class I alleles, at the surface of the cell line used occured, 10 different B-LCL cell lines were incubated with 0 or 150 nM of FL-labeled reference (either HLA-A*0201 or HLA-A*0301) peptide. The FI for each cell line was calculated and the FIs obtained for reference cell lines JY (binding of peptide to HLA-A*0201) and EKR (binding of peptide to HLA-A*0301) were equated to 100% binding. To relate the binding of FL-labeled reference peptide to the 10 different cell lines with the binding of the FL-labeled reference peptide to JY or EKR, the relative peptide-binding percentages were determined. The relative peptide-binding percentages of the FL-labeled reference peptides to each cell line were calculated as: (FI cell line/ FI reference cell line)x 100%. For both FL-labeled reference peptides the non-specific binding to other cell components, of the cell lines used in the competition assay, never exceeded 20% (fig 6). Because the peptide binding motif of HLA-A*0301 is very similar to the binding motif of HLA-A11 (16), binding of the HLA-A*0301 FL-labeled reference peptide to B-LCL cell lines expressing this allele was also observed (fig 6). The cell line *NL* binds the HLA-A*0301 FL-labeled reference peptide. It expresses the HLA-A28 allele of which two subtypes, HLA-Aw6801 and HLA-Aw6803, share the peptide-binding motif with HLA-A*0301 [A. Sette, personal communication].

*Kinetics of peptide-binding to mild-acid treated HLA class I molecules*

To study the effect of peptide binding at different temperatures, EKR cells were eluted and incubated with FL-labeled peptide for different periods of time at 4°C, 26°C or 37°C, respectively. At 4°C the peptide binds initially rapidly and then increases steadily in time (fig. 7). Peptide-binding at 26°C is faster (fig. 7). The amount of peptide bound after 6 hours at 26°C did not differ from the amount of peptide bound at 4°C. Peptide binds fast at 37°C but no increase of bound pept de is found when incubated longer (fig. 7). The lack of increase in bound peptide at 37°C is probably due to two phenomena. The HLA class I molecules, present on the surface of the cell to which no peptide was bound, desintegrate at this temperature (21). Secondly, the dissociation of peptides is dramatically faster at 37°C compared to the dissociation of peptides when incubated at 4°C (22).

*Binding to mild-acid treated class I molecules is not dependent on de novo protein synthesis*

To characterize the interaction of peptides with cell-associated mild-acid treated HLA molecules, peptide stripped EKR cells were incubated with FL-labeled peptide for different periods of time at 4°C or 26°C. As shown in figure 7, the fluorescent labeling at 4°C of the cells steadily increases in time. The use of 100 μM protein synthesis inhibitor emetine for 1 hour prior to elution decreased the amount of peptide bound at 26°C but not at 4°C (fig 7).

Thus, the binding of a peptide to mild-acid treated HLA class I molecules at 4°C was unaffected by the use of a protein synthesis-inhibiting drug. Since metabolic processes are reduced at 4°C, the binding of peptides to the eluted HLA class I molecules is only dependent on the availability of the HLA class I molecules already present at the outer surface of the cell.

*Competition assay*

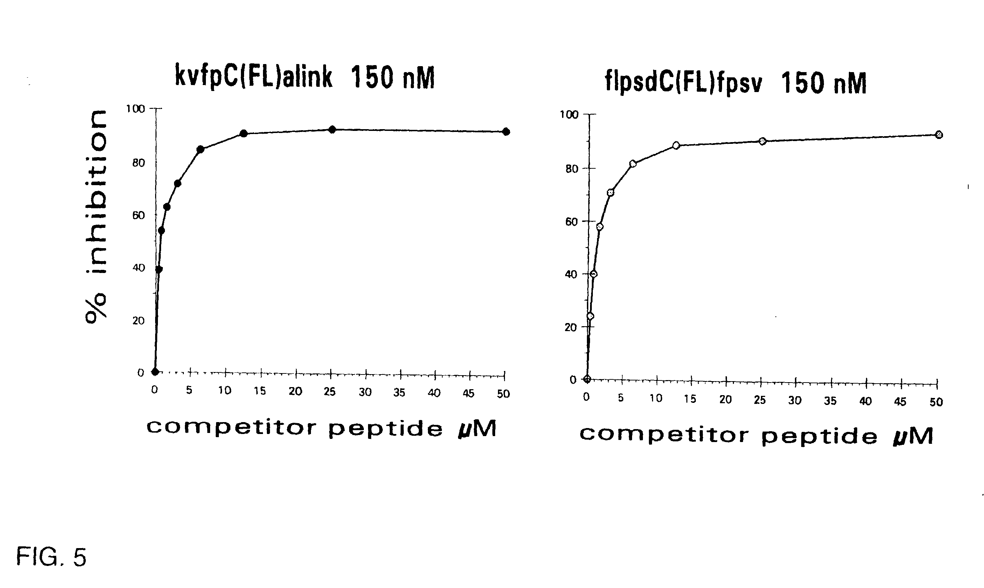

Plotting MF against the concentration of FL-labeled reference peptides resulted in a log-shaped curve. We chose 150 nM of FL-labeled reference peptide as standard concentration in all competition experiments. The use of 150 nM FL-labeled reference peptide resulted in a MF of about 4-5 times the background (not shown). The non-labeled reference peptide was titrated into 150 nM of FL-labeled reference peptide, the percentage inhibition was calculated and plotted against the concentration of the unlabeled peptide (fig 5). In a 24 hour competition assay at 4°C the non-labeled HLA-A*0201 or HLA-A*0301 reference peptide needed about 3-5 times (respectively 0,4 μM and 0,7 μM) the concentration used of the FL-labeled reference peptide to inhibit binding of the FL-labeled peptide to 50% ($IC_{50}$) (Table 1).

To determine the optimal experimental conditions and to validate the assay we tested peptides derived from HPV16 E6 and E7 proteins with known binding properties to HLA-A*0201 or HLA-A*0301 (6, 10) at different concentrations, for 3 or 24 hours at 4°C or 26°C (Table 1). When the cells were incubated for 24 hours less peptide was needed (Table 1). The lowest amount of competitor peptide was needed when the cells were incubated for 24 hours at 4°C (Table 1). No difference was observed between an incubation time of 24 hours or 48 hours at 4°C (not shown). This implicates that the test is more sensitive when equilibrium is reached. Probably, due to a faster association of the FL-labeled reference peptide, more competitor peptide is needed to reach $IC_{50}$ in short incubations. Ranking the peptides to their $IC_{50}$ shows that when the cells are incubated at 4°C for 24 hours, their order is comparable to that found by Kast et al. (6) using the molecular binding assay (Table 1). All peptides that did not possess the described binding motif showed low binding affinity. Taken together these results and the results of peptide-binding to HLA class I molecules on emetine-treated cells, we conclude that the competition assay is best performed at 4°C with an incubation time of at least 24 hours.

*Competition with known CTL epitopes*

Five HLA-A*0201 restricted CTL epitopes, one HLA-A*0301 restricted CTL epitope and two HLA-A*0301 peptides, identified via peptide pool-sequencing, were used to determine the $IC_{50}$-values of high affinity binding peptides. The five peptides tested for binding to HLA-A*0201 all competed very well with an $IC_{50} \leq 1.7$ μM (Table 2). The known HLA-A*0301 restricted CTL epitope derived from HIV was tested. This peptide, derived from HIV-nef, bound with an $IC_{50}$ of 0.5 μM. The two peptides, which were identified via peptide pool sequencing bound with an $IC_{50} \leq 15$ μM (Table 2). We therefore conclude that peptides competing with an $IC_{50} \leq 5$ μM must be considered potential CTL epitopes.

*Binding of conserved HIV-1 pol sequences to HLA-A*0301*

Twenty peptides of 8-11 amino acids long were selected on the basis of the HLA-A*0301 binding motif and their conservation in the polymerase gene products of different HIV-1 strains. The peptides were tested in the competition assay for 24 hours at 4°C. Nine peptides were shown to bind to HLA-A*0301. Four peptides bind with intermediate-affinity and competed with an $IC_{50} \leq 5$ μM (Table 3), the other five peptides (marked with an asterisk; *) bind with high affinity and competed with an $IC_{50} \leq 3.0$ μM. Considering the $IC_{50}$ obtained with the known CTL epitopes, especially these five peptides may be candidate CTL epitopes.

Comments

For an extensive discussion of these results see (3). This example shows that this assay performs well with respect to peptides of known binding capacity to either HLA-A*0201 or HLA-A*0301. The kinetics of peptide binding in this assay were shown to be comparable to that in assays employing soluble HLA class I molecules. Furthermore, application of the assay in the search for potential HLA-A*0301 restricted CTL epitopes, derived from HIV-1 polymerase, resulted in the identification of five high-affinity binding peptides. The assay is easy to perform because there is no need to purify HLA class I molecules, or to transfect cells with HLA class I molecules and no radioactive label is used. Moreover, large panels of HLA-typed human B-cell lines are available, as tools for peptide-binding to a vast array of HLA molecules. Presently, the system is also used succesfully for the identification of peptides that bind to HLA-A*0101 and HLA-B7.

Legends to Figures Example 1

*Figure 1 . Specificity of FL-labeled reference peptides.*

Reference cell line EKR (HLA-A*0301) was mild-acid treated at pH=2.9. The reference cell line JY (HLA-A*0201) was mild-acid treated at pH=3.2, and the 10 different other B-LCL lines were mild-acid treated at pH=2.9, when subjected to incubation with the HLA-A*0301 FL-labeled reference peptide, or at pH=3.2 when incubated with the HLA-A*0201 FL-labeled reference peptide. EKR cells are incubated with 150 nM of the HLA-A*0301 FL-labeled reference peptide (*open bars*), JY cells are incubated with 150 nM of the HLA-A*0201 FL-labeled reference peptide (*hatched bars*) and the 10 different other B-LCL lines were incubated with 150 nM of either the HLA-A*0301 (*open bars*) or HLA-A*0201 FL-labeled reference peptide (*hatched bars*), for 4 hr at 26°C. The fluorescence index (FI) was calculated for each cell line and the FI of FL-labeled reference peptide bound to EKR (for binding to HLA-A*0301) and the FI of FL-labeled reference peptide to JY (for binding to HLA-A*0201) was equated to 100% binding. By the formula: (FI cell line/FI reference cell line)*100% the relative peptide-binding percentages of the 10 different B-LCL lines was calculated. The upper left side shows the full HLA-type of the reference cell lines together with the overlapping HLA-type of other cell lines. The lower left side shows all 10 B-LCL lines with their full HLA-type.

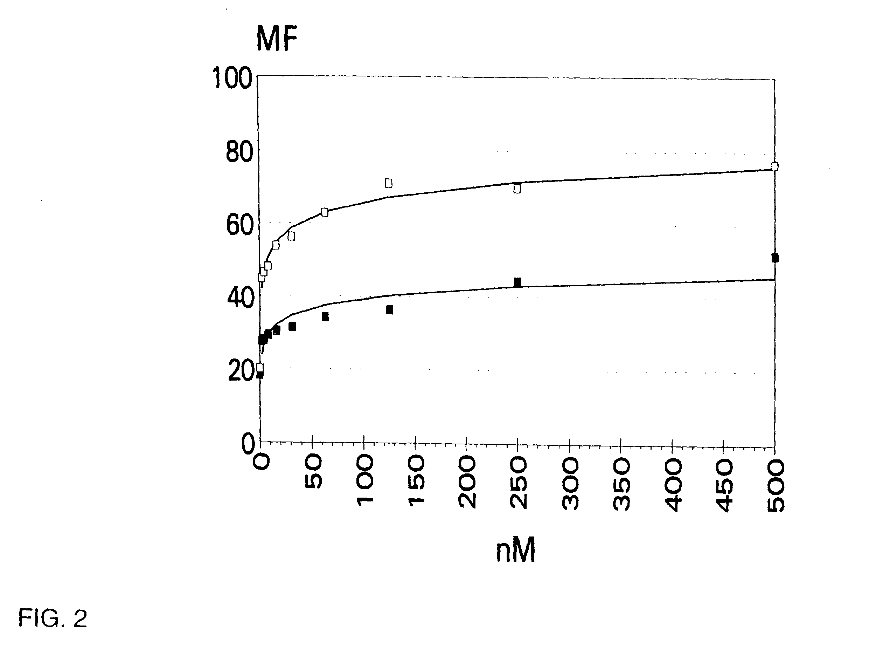

*Figure 2 . Peptide binding on eluted vs not-eluted HLA class I molecules.*

JY cells (*closed symbols*) and JY cells of which their HLA class I molecules were mild-acid treated (*open symbols*), were incubated with increasing amounts (nM) of the HLA-A*0201 FL-labeled peptide. Cells were incubated for 3 hours at 26°C, washed and mean-fluorescence (mF) was measured at a FACScan. The lines shown are the result of logarithmic regression analysis of the concentration of FL-labeled reference peptide versus the mF.

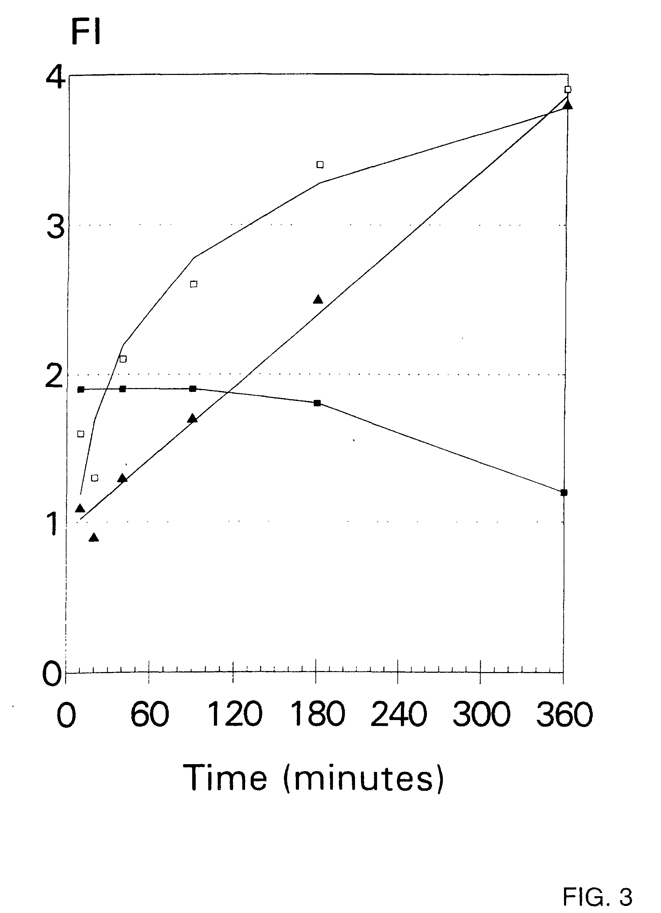

*Figure 3 . Kinetics of peptide binding to mild acid treated HLA class I molecules.*

EKR cells were mild acid treated and incubated with 150 nM of HLA-A*0301 FL-labeled reference peptide for different periods of time at 4°C (*triangles*), 26°C (*open squares*) or 37°C (*closed squares*). At 10, 20, 40, 90, 180 and 360 minutes the binding of Fl-labeled peptide was measured. Binding is given as the fluorescence index (FI). The lines shown for 4°C and 26°C are the result of respectively lineair or logarithmic regression analysis.

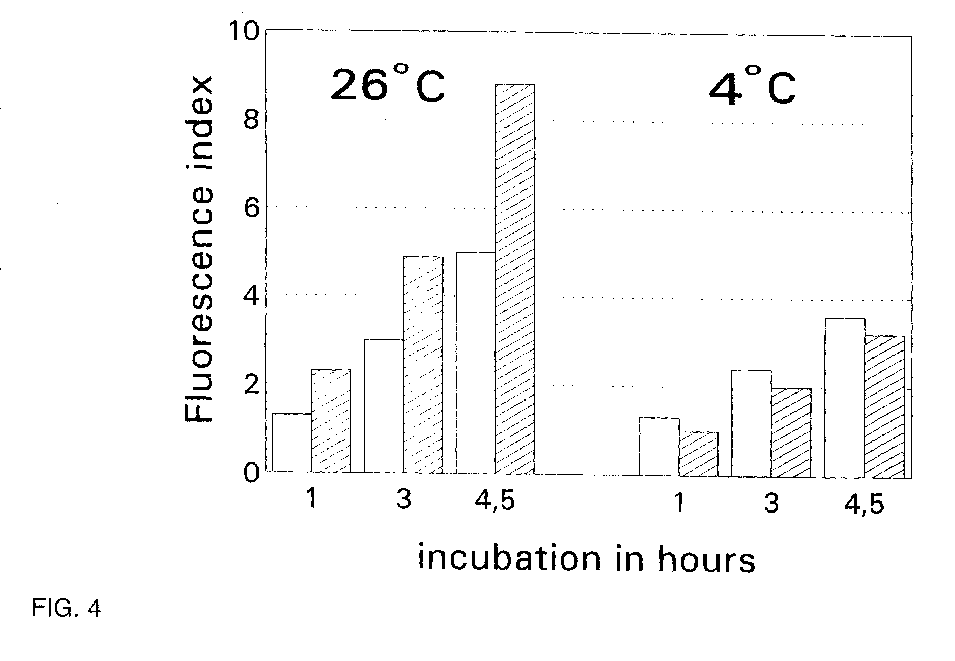

*Figure 4. Binding of FL-labeled peptide to protein synthesis inhibiting drug treated cells.*

EKR cells were treated with $10^{-4}$M emetine (*open bars*) or not (*hatched bars*), for 1 hour prior to mild-acid treatment (20). 150 nM of HLA-A*0301 FL-labeled reference peptide was added and binding was monitored at 1, 3 or 4.5 hours of incubation. Cells were incubated at 26°C or 4°C.

*Figure 5. Competition of non-labeled reference peptide with FL-labeled reference peptide.*

EKR cells (left) or JY cells (right) were incubated with 150 nM of FL-labeled reference peptide, kvfpC(FL)alink or flpsdC(FL)fpsv respectively, and increasing amounts (µM) of non-labeled reference peptide. Inhibition of binding was calculated and showed in relation to the amount of non-labeled reference peptide used.

**§4.2 An HLA class I peptide-binding assay that measures the stability of peptide-MHC complexes at the surface of intact human B cells.**

The binding affinity of peptides to MHC molecules at equilibrium is the resultant of the continued association and dissociation of the tri-molecular complex of peptide, MHC class I molecule and β2m. The dissociation rate of peptides bound to MHC class I, is neither influenced by the presence of competing peptides (23) nor by the concentration of the competing peptides (24). On the other hand, the amount of free MHC peptide binding sites is influenced and limited by the dissociation rate of previously bound peptide (24). Thus a peptide with a low dissociation rate will, once bound, probably form a stable MHC-peptide complex in the ER, be transported to the cell-surface and persist there for a time sufficient to allow T-cell recognition.

In order to investigate the correlation between stability of the peptide-MHC complex and immunogenicity we have determined the dissociation rate of a group of MHC class I binding peptides. This assay measures the stability of peptide-MHC complexes at the surface of intact HLA-homozygous B-cells. Comparison of the correlation between immunogenicity and peptide binding affinity on one hand and between immunogenicity and the dissociation rate of peptide from MHC class I molecules on the other hand has shown that immunogenicity correlates better with the dissociation rate than with peptide binding affinity.

As this complex-stability assay makes use of intact human B cells, it shares the advantages described for the peptide binding affinity assay (see §4.1).

**Example 2.**

**Immunogenicity of peptides bound to MHC class I MHC molecules correlates well with stability of the MHC-peptide complex.**

Material and Methods

*Cell lines*

The EBV transformed B-cell line: JY (HLA type:A*0201, B7, Cw7, DR4, DRw6, DPw2) was cultured in complete culture medium consisting of RPMI 1640 Dutch modification (Gibco BRL, Paisley, Scotland) supplemented with 10% FCS, antibiotics (100 IU/ml penicillin (Brocades Pharma, Leiderdorp, The Netherlands) and 100 ug/ml kanamycin (Sigma, St. Louis, MO, USA)), and 20 µM 2-ME (Merck, Darmstadt, Germany) at 37°C in humidified air containing 5% $CO_2$.

Jurkat_A*0201K$^b$ cells are stable transfectants of the human T cell leukaemia line, Jurkat, which express the product of the HLA-A*0201K$^b$ chimeric gene (25). They are cultured in complete culture medium in the presence of 200ug/ml G418 sulphate.

*Peptides*

Peptides were synthesized by solid-phase strategies on an automated multiple peptide synthesizer (Abimed AMS 422, Langenfeld, Germany) using Fmoc-chemistry. Peptides were analyzed by reverse phase HPLC, dissolved in 20 µl DMSO, diluted in 0.9% NaCl to a peptide concentration of 5 mg/ml and stored at -20°C before usage.

Fluorescein (FL)-labeled peptides as used in the competition based HLA class I binding-assay were synthesized, labeled and characterized as described earlier (3). The sequence of the reference peptide used for HLA-A*0201 was FLPSDYFPSV (14) wherein we substituted the tyrosine with a cysteine to tag a fluorescein group to the peptide: FLPSDC(FL)FPSV (3).

*Transgenic mice*

HLA-A*0201K$^b$ transgenic mice were kindly provided by Dr L. Sherman (Scripps Laboratories, San Diego, USA; through animal distributor Harlan Sprague Dawley, Inc., Indianapolis, USA). Mice were held under clean conventional conditions. The transgenic mice express the product of the HLA-A*0201K$^b$ chimeric gene in which the a3 domain of the heavy chain is replaced by the corresponding murine H-2 K$^b$ domain while leaving the HLA-A*0201 a1 and a2 domains unaffected (25). This allows the murine CD8 molecule on the murine CD8+ T cells to interact with the syngeneic a3 domain of the hybrid MHC class-I molecule.

*In vivo immunizations and murine T cell cultures*

Groups of 3-6 HLA-A*0201K$^b$ transgenic mice were injected subcutaneously in the base of the tail with 100ug peptide emulsified in IFA in the presence of 140ug of the H-2 I-A$^b$-restricted HBV core antigen-derived T helper epitope (128-140; sequence TPPAYRPPNAPIL) (26). After 11 days, mice were sacrificed and spleen cells ($30 \times 10^6$ cells in 10 ml) were restimulated *in vitro* with syngeneic irradiated LPS-stimulated B cell lymphoblasts (ratio 3:1), and 1 ug/ml peptide in complete culture medium in T25 flasks (Falcon, New Jersey, USA). At day 6 of culture, the cytotoxicity of these bulks was tested in a standard 5 hour $^{51}$Chromium ($^{51}$Cr) release assay.

*$^{51}$Cr Cytotoxicity assay*

CTL activity was measured in a standard chromium release assay as described previously (27). Target cells were sensitized with 10 ug/ml peptide for 30' at 37°C. Target cells were added to various numbers of effector cells in a final volume of 100 µl of complete culture medium in 96-wells U-bottom microtiter plates. After 5 hours of incubation at 37°C, supernatants were harvested. The mean percentage specific lysis of triplicate wells was calculated as follows:

% specific lysis = ((experimental release-spontaneous

release) / (maximal release-spontaneous release)) x 100

Percentage specific lysis is expressed in LU30%/$10^6$cells, in which 1 LU30% corresponds to the number of effector cells required to induce 30% $^{51}$Cr release from 2000 Jurkat A*0201/$K^b$ target cells during a 5-h assay.

*Peptide 'stripping' by mild-acid treatment and competition based HLA class I peptide-binding assay*

See Example 1

*Measurement of MHC-peptide complex stability at 37°C*

JY cells at a concentration of 1-2 million cells/ml were incubated with $10^{-4}$M emetine (Sigma, St. Louis, USA) for 1 hour at 37°C to stop protein synthesis and thus the emergence of *de novo* synthesized class I molecules at the cell-surface (20). Cells were washed twice with PBS and peptide-stripped (see above). One million cells were added to 200ug peptide in 1 ml and incubated for 1 hour at room temperature. Cells were washed twice with ice-cold IMDM and resuspended in 1 ml IMDM. Subsequently, the cells were incubated for 0, 2, 4 and 6 hours at 37°C and thereafter stained with BB7.2, an HLA-A2 conformation specific monoclonal antibody (28) and GaM/FITC. Thereafter the cells were fixed by resuspension in PBA1% containing 0.5% paraformaldehyde. Cells were analyzed by FACscan. The fluorescence index (FI) was calculated as FI= (mean fluorescence sample - mean fluorescence background) / mean fluorescence background (without peptide). Samples were tested in duplo and the variation between both samples was allways less that 10%.

The percentage of residual HLA-A2 molecules was calculated by equating for each peptide, the FI of t=0 to 100% and then use the formula: %remaining= ($FI_{t=n}$ / $FI_{t=0}$) x 100%. As the dissociation of peptides from MHC is a linear process, the stability of the peptide-MHC complexes was measured as the time required for 50% of the molecules to decay (DT50%). We've used t=2 hours at 37°C as starting point for the reason that from this time point only the DT50% are determined from peptides that are able to form stable peptide-MHC complexes.

*Statistics*

Using the Fisher's test for 2 by 2 tables (Fisher's exact 2-tailed test), the dissociation rate (DT50%) of peptides at 37°C was correlated to the immunogenicity of the peptides. Binding-affinity could not be correlated to immunogenicity using a Chi-square test due to the relatively small number of peptides. Therefore we compared high affinity binding peptides with low affinity binding peptides in order to establish the strongest correlation between affinity and immunogenicity using the Fisher's test for 2 by 2 tables.

Results

*Stability of MHC class-I molecules complexed with HBV or HPV16 derived peptides of known binding affinity and immunogenicity in HLA-A*0201/$K^b$ transgenic mice*

To study the relation between dissociation of peptides bound to MHC class-I molecules and their ability to induce a CTL response, we used 9 peptides derived from HBV polymerase (pol) and 8 peptides of HPV16 of which the relative binding affinity and immunogenicity in HLA-A*0201/$K^b$ transgenic mice was reported previously (6, 10, 29). To show that all 17 peptides indeed bound to HLA-A*0201 we tested their affinity in a previously described competition based HLA-class I binding-assay (3). HBV*pol*-635, HPV16*E7*-11 and HPV16*E7*-86 bound with relatively high affinity (< 5 µM). Fourteen peptides bound with intermediate (between 5 and 15 µM) or low affinity (> 15 µM; Table I). Peptide binding affinities measured and classification of the peptide binding affinity into high, intermediate and low are comparable to the affinities and classifications of Sette et al. (10) and Kast *et al*. (6).

Subsequently with the use of a conformation specific anti-HLA-A2 antibody, the amount of residual HLA-A*0201 peptide complexes was monitored in time. The loss of peptide-stabilized HLA-A*0201 molecules at the cell-surface represents the dissociation of the peptide from the class-I molecule to which the peptide is bound. The stability is then presented by the time required for 50% of the molecules to decay (DT50%). All three high affinity binding peptides and three of the intermediate affinity binding peptides, HBV*pol*-996, HBV*pol*-1076 and HPV16*E7*-82 showed a DT50% of more than 3 hours (Table I). The four other peptides of intermediate affinity, HBV*pol*-1344, HPV16*E6*-18, HPV16*E6*-52 and HPV16*E7*-7 showed a DT50% between 1 and 2 hours (Table I). The low affinity binding peptides showed a DT50% of 1 hour or less. In Table II we show a comparison between the dissociation rate, binding affinity and immunogenicity of these peptides. All high affinity binding peptides form stable MHC-peptide complexes and are immunogenic, whereas the group of peptides of intermediate affinity contains either peptides that are immunogenic and form stable MHC-peptide complexes or are non-immunogenic and do not form stable MHC-peptide complexes as shown by their high dissociation rates (Table II).

*Stability of MHC class-I molecules complexed with known human CTL epitopes*

Seventeen HLA-A*0201 binding peptides earlier reported to be immunogenic (e.g. found as CTL epitope or capable of inducing a primary response) (6, 12, 27, 30-40) were tested for their binding affinity to HLA-A*0201. Eight peptides bound with high affinity, 7 peptides bound with intermediate affinity and 2 peptides bound with low affinity (Table III). The dissociation rates were determined and virtually all peptides showed a DT50% > 4 hours, except for the peptides HPV11*E7*-4 and HIV-1*pol*-267. The HPV11*E7*-4 and HIV-1*pol*-267 CTL epitopes, both found by primary CTL induction using synthetic peptide or cells expressing extremely high amounts of antigen, dissociated faster (DT50% > 2 hours ; Table III). Interestingly, the sequence of the HCV1*core*-131 peptide [ADLMGYIPLV] does not correspond precisely to the HLA-A*0201 motif. The HCVcore-132 peptide which lacks the N-terminal alanine [DLMGYIPLV] fits better to the HLA-A*0201 motif. This is also reflected in the higher affinity of this shorter peptide ($IC_{50}$=5.0 μM) but the peptide dissociates dramatically faster (Fig 1.) than the HCV*core*-131 peptide.

*Immunogenicity is correlated with the dissociation rate*

A significant correlation exists between the immunogenicity of a peptide and the dissociation rate. Of the investigated known HLA-A*0201-restricted immunogenic peptides, 21 out of 23 showed a DT50% > 3 hours, while none of the 11 non-immunogenic peptides showed a DT50% >3 hours (p=0.0000003, Table IV). This correlation is closer than that between peptide binding affinity and immunogenicity (p=0.0005, Table IV) and confirms the trend visible in Table II. When the correlation between immunogenicity and dissociation rate was investigated for peptides binding with intermediate or low affinity, this was still better correlated (p=0.00007, Table V) to immunogenicity than affinity (p=0.04). This implies that peptides that are processed, transported to the endoplasmic reticulum and are able to form stable MHC-peptide complexes are likely to be CTL epitopes.

*Immunogenicity in HLA-A*0201/$K^b$ transgenic mice of HIV-1 derived peptides with known affinity and dissociation rate*

To assess the in vivo immunogenicity of peptides of which the binding affinity and the dissociation rate was measured, HLA-A*0201/$K^b$ transgenic mice were vaccinated with two control peptides (HPV16*E7*-86 and HBVcore-18; FLPSDDFPSV) and four HIV-1 derived peptides (Table VI). The derivation of these transgenic mice (25) and their use to analyze in vivo immunogenicity have been described previously (10, 29). The HIV-1*pol*-468; (ILKEPVHGV) is a CTL epitope and binds with intermediate affinity. The HIV-lpol-267;(VLDVGDAYFSV) peptide was found to be immunogenic in a human primary CTL induction after repetitive stimulations with relatively high doses of peptide (27). To test the predictive value of the *in vitro* measured MHC-peptide complex stability we determined the binding-affinity and dissociation rate of the two other HIV-1*pol* peptides (HIV-1*pol*-343: YMDDLYVGSDL and HIV-1*pol*-576: LLWKGEGAV) (Table VI). Both peptides were detected when the highly conserved regions of HIV-1*pol* were screened for amino acid sequences that contained two anchors for binding to HLA-A*0201, as described previously (27). We vaccinated groups of mice with all the peptides. Bulk CTL derived from mice vaccinated with the control peptides specifically lysed peptide-sensitized Jurkat A*0201/$K^b$ cells (Fig. 2; Table VI). As expected, all peptides with a low dissociation rate mounted a CTL response (Fig. 2 ; Table VI), whereas the two peptides with high relative dissociation rates did not induce a CTL response (Fig. 2; Table VI). Thus, the immunogenicity of these peptides was perfectly predicted by their dissociation rates.

Comments

This example shows that the measurement of MHC-peptide complex stability is highly valuable in identifying potential T cell epitopes. Newly defined immunogenic peptides formed relatively stable MHC-peptide complexes as shown by their low dissociation rates, whereas non-immunogenic peptides displayed high dissociation rates. In addition, virtually all previously described HLA-A*0201 restricted T cell epitopes showed low dissociation rates. Furthermore, we show that the immunogenicity of HIV-1 derived peptides can be predicted more accurately by their dissociation rate than by the MHC class I binding affinity. We find a closer correlation between the dissociation rate of a peptide and immunogenicity (p=0.0000003) than between binding affinity and immunogenicity (p=0.0005). The better correlation is gained in the group of peptides that bind with intermediate or low affinity. In conclusion, selection of peptides based on affinity and their dissociation rate leads to a more precise identification of candidate CTL epitopes than selection based on affinity alone.

Note that this assay requires HLA-type specific MAbs that can discriminate between empty and peptide-loaded molecules. Although such Abs are currently available for HLA-A*0201 and -A*0301, additional Abs need to be identified or isolated for defining the stability of peptide-MHC complexes in the context of other HLA class I molecules. Approaches to isolate such Abs include:

* Screening of available Abs (ATCC, other laboratories) for desired characteristics
* Selection of appropriate Abs against a human B cell line expression the relevant HLA-molecule from a semi-synthetic phage antibody display library (in collaboration with Dr. T. Lochtenberg, University Hospital Utrecht, The Netherlands; (41)).
* Generation of monoclonal Abs, or selection of phage antibodies against purified, peptide-loaded MHC molecules (6).

Legends to Figures Example 2

*Figure 6. Binding affinity and dissociation rate of the HCV1core-131 peptide and the shorter variant without the N-terminal alanine.*

The binding affinity (left) and the dissociation rate (right) of the HLA-A*0201 restricted CTL epitope HCV1*core*-131 [*closed symbols*; ADLMGYIPLV] (31) and shorter variant [*open symbols*; DLMGYIPLV], which corresponds more precisely to the HLA-A*0201 motif, was tested (see material & methods). The mean inhibition of the reference peptide at each concentration of competitor peptide, obtained in two independent experiments, is shown at the left. The right figure shows the percentage of residual peptide-MHC molecules for both peptides at each time-point (mean of two independent experiments). The percentage of molecules present at t=2 hours was set to 100%. The lines are the result of linear regression analysis.

*Figure 7. Peptide-specific cytotoxicity induced by vaccination of HLA-A*0201K$^b$ transgenic mice.*

A representative experiment in which HLA-A*0201K$^b$ transgenic mice were vaccinated with indicated peptide displaying a low dissociation rate (A,B,E) or high dissociation rate (C,D) in combination with an HBV core-encoded T helper epitope in IFA (see material and methods). Bulk CTL cultures derived from spleen cell of these mice were tested for peptide specificity in cytotoxicity assays on Jurkat A*0201K$^b$ target cells pulsed with (*open symbols*) or without (*closed symbols*) specific peptide. Shown is the mean specific lysis of bulk CTL from 3-6 animals with indicated standard deviation. Specific lysis is depicted at E/T ratio varying from 1.5 to 100.

**Example 3**

**Identification of melanoma associated immunogenic peptides using an assay that measures stability of the MHC-peptide complex.**

Materials and methods

Most procedures have been described in Examples 1 and 2. Induction of CTL by stimulating human T lymphocytes with peptide-loaded dendritic cells (DC) was performed as follows: Monocyte-enriched Human Peripheral Blood Monocyte (PBMC) fractions were isolated by plastic adherence of total PBMC from HLA-A*0201-subtyped healthy donors. Adherent cells were cultured for 5-7 days with RPMI/Lglutamine/antibiotics/10% FCS or 10% human serum (HS), and 500 U/ml rHuIL-4, and 800 U/ml rHuGM-CSF. Culture medium with cytokines was replenished every other day. Cultures were treated for 24 h with 50 U/ml rHuIL-1a and 200 U/ml g-IFN, and pulsed with 50 ug/ml peptide in RPMI/L-glutamine/antibiotics/1% FCS for 4 h. Peptide-pulsed stimulators were irradiated (2500 Rads) and washed twice. In each well of a 24-well plate 1 ml of RPMI/L-glutamine/antibiotics/5% HS was dispensed containing 1-2x10$^4$/

ml stimulator cells.

Autologous responder cells were enriched for (CD8+) T-cells by adherence to plastic dishes, followed by depletion of CD4+ cells using Dynabeads (Dynal, Olso, Norway). Total PBMC responders were mixed with the CD8-enriched non-adherent cells, to bring the final responder population to approximately 10% CD4+ T cells. Responders were mixed with stimulators in a 1:10 to 1:20 ratio, to a total of $2x10^6$ responders per well. rHuIL-7 was added to 5 ng/ml. Medium + rHuIL-7 was replenished after 7 days. At day 12, responders were restimulated with autologous peptide-pulsed adherent PBMC (as described previously: (42)). rHuIL-2 was added to a final concentration of 120 IU/ml. Similarly, CTL cultures were restimulated weekly. CTL cultures were subcloned in U-bottom 96-well plates by limiting dilution, using the HLA-A*0201+, MelanA/MART-1 expressing FM3 Melanoma cell line (5000/well; (43)), and a mixtures of allogenic PBMC from six donors (100.000/well) and three HLA-A*0201$^+$ B-LCL (5000/well), in RPMI/Lglutamine/antibiotics/5% HS + 120 IU/ml rHuIL-2. Clones were restimulated weekly.

Results

The melanoma antigen Melan-A/MART-1 was screened for the presence of potential HLA-A*0201-binding CTL epitopes using three peptide binding assays: the T2-binding assay (2), a binding assay that uses HLA-A*0201-molecules on intact human B cells (see Example 1), and an assay that measures the stability of the MHC-peptide complexes (see Example 2). Comparison of the binding-data (see Table IX) shows that the nonamer peptide AAGIGILTV, which represents a previously described immunodominant peptide-epitope presented by HLA-A*0201-positive melanoma cells (44), binds poorly to T2 cells and only shows modest binding to HLA-A*0201 on intact human B cells. The 10-mer variant of this peptide (EAAGIGILTV), however, displays considerable binding to HLA-A*0201 in both binding assays. Paradoxically, comparison of these two peptides with respect to the stability of peptide-MHC complexes shows that the 9-mer peptide, when bound to HLA-A*0201, forms stable peptide-MHC complexes, whereas complexes with the 10-mer peptide are unstable.

Peptide-specific CTL immunity was raised in vitro by stimulating peripheral blood lymphocytes of HLA-A*0201-positive healthy donors with autologous dendritic cells that were loaded with either of the two peptides. The reactivity of the resulting CTL was tested against T2 cells loaded with the relevant peptides as well as to HLA-A*0201- and MART-1-positive human melanomas cells. These experiments clearly demonstrated that tumor-specific CTL activity that reacted against both peptide-loaded T2 cells and melanoma cells was only obtained after stimulation of the donor lymphocytes with the 9-mer peptide AAGIGILTV (these experiments will be described elswhere; Van den Elsas et al., manuscript in preparation). These data clearly demonstrate that immunogenicity of a peptide epitope correlates strongly with the stability of the corresponding peptide MHC complex, whereas MHC-binding of a peptide as measured on T2 cells or intact B cells does not ensure that this peptide (i) will form stable MHC-peptide complexes and (ii) is immunogenic found to show strong and stable binding to HLA-A*0201 in all three assays (see Table IX). Also against these two peptides CTL reacting against both peptide-loaded T2 cells and HLA-A*0201-/MART-1 positive melanoma cells could be raised (Van den Elsas et al., manuscript in prep.).

Taken together these results show that selection of immunogenic peptides based on stability of the MHC-peptide complex is a valuable tool in the identification of tumor-associated T cell epitopes.

**§4.3 Identification of immunogenic peptides**

The present invention provides an novel technique for identifying MHC-binding peptides that can serve as a target for an immunotherapeutical T cell response. This method will be applied in conjunction with other selection steps (see §1) to screen the primary sequence of proteins that are expressed by for instance tumors for peptides that are likely to be processed and presented by tumor cells and that will constitute an immunogenic target for the T cell immune system.

Peptide-epitopes derived from the following antigens are included in our studies:

* E6-protein of human papilloma virus type 16 and 18 (HPV16, HPV18)
* E7-protein of human papilloma virus type 16 and 18 (HPV16, HPV18)
* Gag, Pol and Env-proteins of human immunodeficiency virus (HIV)
* MAGE-2 human melanoma antigen
* Tyrosinase human melanoma antigen
* Melan-A/MART-1 melanoma antigen
* p21Ras human onco-protein
* p53 human onco-protein
* human carcino-embryonic antigen (CEA)
* human epithelial cell adhesion molecule (EpCAM)

* CD19 human B cell-specific protein
* CD20 human B cell-specific protein
* CD44 cell surface glycoprotein
* The immunoglobulin (Ig) variable domains of the Ig heavy and light chains expressed by B cell lymphomas

The sequences of the proteins mentioned above are screened for peptides that are likely to represent immunogenic T cell epitopes in the context of the following HLA class I molecules:

* HLA-A*0101
* HLA-A*0201
* HLA-A*0301
* HLA-A*1101
* HLA-A*2401
* HLA-B7

### § 4.4 List of peptides screened in a stability assay because they have been through the preselection procedures.

As illustrated in § 4.3 Examples 2 and 3, selection of immunogenic peptides is greatly improved in accuracy when peptides are screened not only for binding to the MHC molecules concerned, but also for the stability of the resulting peptide-MHC complexes. In previous publications we have described multiple potential immunogenic peptides derived from various (e.g.tumor-)antigens. These peptides were selected on the basis of a two-step procedure, consisting of (i) computer-prediction and (ii) binding assays that do not take into account the stability of peptide-MHC complexes. The person skilled in the art can now apply, and thereby further validate, our novel assay for measuring peptide-MHC complex stability with respect to these peptides. A list of these peptides is provided in Tables X - XX.

### § 4.5 Vaccination with recombinant adenoviruses harbouring several defined T cell epitopes in string-of-bead constructs.

T cell-mediated immunity to viruses or tumors can be induced in two ways: passive, by transfer of virus- or tumor specific T cells, or active, by exposure to antigen. In the latter case, antigen can be given to the host in many different forms, ranging from whole attenuated viruses or tumor cells to isolated proteins. In virtually all these cases the vaccines are not rationally designed in the sense that the minimal essential T cell epitopes are known. Therefore, immunization in these cases may not always leas to the desired effect. For example, immunization with attenuated viruses, like vaccinia, may induce unwanted side-effects or result in T cell immunity to epitopes that are subjected to antigenic variation by the wild-type virus. Likewise, immunization with a single protein can be ineffective, because it may induce only T cell-responsiveness to the immunodominant T cell epitopes, without inducing T cell-responses to other, subdominant T cell epitopes, or it may not contain sufficient CTL epitopes to cover the whole target population. In part, these disadvantages can be overcome by exploiting other vaccination strategies.

Vaccination strategies using recombinant viruses expressing the antigens of choice are currently under development. In the case of the development of anti-tumor vaccines, several tumor-associated antigens, like MART1 and gp100 are good candidates for the incorporation into a recombinant viral vector. However, the delivery of whole genes encoding tumor-associated antigens by recombinant viral vectors as a way to evoke anti-tumor immunity might be unsafe when these tumor-associated antigens are involved in carcinogenesis. For example, viral vector vaccines for treatment and prevention of HPV16-positive cervical carcinoma are intrinsically hazardous if such vaccines contain the functional human papilloma virus type 16 (HPV16) E6 and E7 oncogenes. The same holds true when the viral vector encodes the oncoprotein HER2/neu, cyclin-dependent kinase 4, the aberrant fusion proteins BCR-ABL or mutated Ras and p53 proteins, because these genes are implicated in the development of cancer. Likewise, incorporation of the genes belonging to the family of tumor-associated antigens MAGE, GAGE or BAGE into viral vectors should be avoided, because their function has until now not been identified. However, by introducing only the sequences that encode T cell epitopes derived from such tumor-associated antigens into recombinant viral vectors it should be feasible to direct the immune response to those targets without introducing potential hazards as transformation of somatic, vector infected cells.

Recently, studies have been reported that describe the successful use of a recombinant vaccinia vaccine expressing several CTL epitopes in a string-of-bead fashion in mice (48), (49). These studies show the potency of the use of string-bead-vaccines for the induction of anti-viral and anti-tumor immunity. However, due to the potential risks associated with vaccinia and the decreasing or absent (in younger individuals) immunity to poxvirus due to the abolished vaccination programme with poxvirus, recombinant vaccinia vaccines cannot be used in humans. Moreover, in these studies the CTL epitopes were directly linked to each other, and did not contain spacer-sequences that direct efficient and accurate processing and presentation of the CTL epitopes. For these reasons, rAd harbouring several CTL epitopes in a string-of-bead fashion with proteolytic cleavage sites between the CTL epitopes leading to optimal processing and presentation of the incorporated CTL epitopes will induce stronger CTL responses without inducing harmfull side-effects.

Recombinant adenovirus, harbouring whole tumor-associated antigens, have been used to induce protective anti-tumor immunity (50-52).(53, 54), illustrating the possibility to use rAd for the induction of tumor-specific protective immunity.

By incorporation of minigenes containing multiple-T cell-epitopes and proteolytic cleavage sites in between these T cell epitopes into a rAd we now have developed a novel and innovative method for the induction of protective T cell responses against viruses and tumors.

**Example 4.**

**An rAd expressing several defined CTL epitopes in a string-of-bead fashion induces protective anti-tumor immunity.**

Materials and methods

Cell lines

Mouse embryo cells (MEC), Ad5E1 transformed MEC, Ad5E1 + ras transformed MEC, HPV16-transformed MEC, COS-7 cells were maintained in Iscove's modified Dulbecco's medium (Biocrom KG, seromed, Berlin, Germany) supplemented with 4% FCS (hyclone laboratories , Logan, Utah), penicillin, (110 IU/ml; Brocades Pharma, Leiderdorp, the Netherlands), and 2-mercaptoethanol (20 μM) at 37°C in a 5% $CO_2$ atmosphere. CTL clones were cultured as described elsewhere (55, 56), (1057 The influenza matrix-specific HLA-A*0201-restricted CTL clone was grown on HLA-A*0201-positive EBV-transformed B cell lines irradiated with 30 Gy in RPMI. 911 cells were grown as described in (58).

Generation of rAd

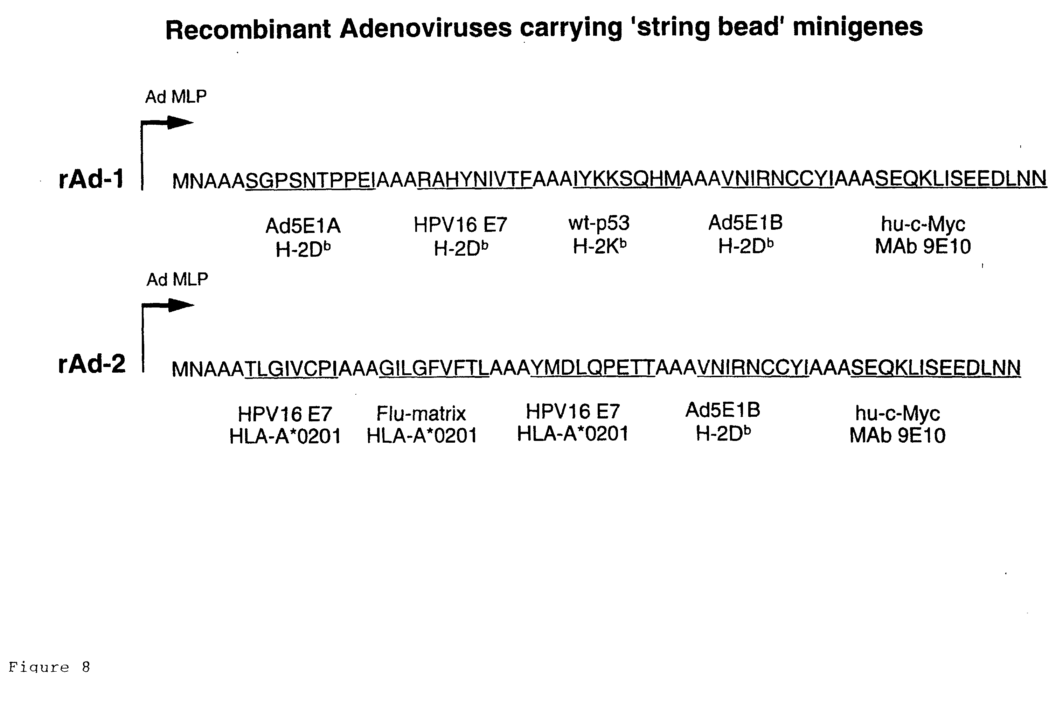

Minigene 1 or minigene 2 (see Fig 8) were inserted into the shuttle vector pMad5. pMad5 (R. Hoeben, unpublished) was derived from pMLP10 (73) through the following cloning steps: (i) deletion of the SalI/BamHI-fragment, (ii) insertion of a polylinker sequence (ClaI, MluI, SnaBI, SpeI, AsuII, MunI) into the unique Hind III site, directly downstream of the Ad5 major late promoter (MLP) and Ad2 tripartite leader sequences, (iii) Insertion into the MunI site of a BglII/XhoI fragment of the Ad5 genome, which permits homologous recombination of the pMad5 sequences with sequences of pJM17 (see below). Insertion of minigenes 1 and 2 was performed in two steps. First pMad5 was cleaved with enzymes SpeI and MluI and the 5'ends were dephosphorylated. The annealed and phosphorylated double-stranded oligonucleotides 1a/b and 2a/b (see Table A) were ligated into this vector, which resulted in a small open reading frame consisting of a methionine, a spacer with the sequence NASYATS and the human c-myc sequence SEQKLISEEDLNN. The latter sequence corresponds to an epitope which can be recognized by the appropriate monoclonal antibody (74). As a result of the cloning strategy, the original SpeI and MluI sites of pMad5 were destroyed, whereas new SpeI and MluI sites were created between the Start codon and the c-myc epitope encoding sequence. In a second cloning step the CTL epitope encoding sequences were inserted into the cassette. The cassette vector was cleaved with enzymes SpeI and MluI and the annealed non-phosphorylated double-stranded oligonucleotides 3a/b and 4a/b were ligated into the open vector (minigene 1). Alternatively, the annealed non-phosphorylated double stranded oligonucleotides 5a/b and 4a/b were ligated into the open vector (minigene 2). Subsequently, the non-ligated oligonucleotides were removed from the ligation mixture by Sephacryl 400 column-purification. The eluted DNA was added to a ligation-reaction that contained the annealed and phosphorylated double-stranded oligonucleotides 6a/b and 7a/b (minigene 1), or phosphorylated double-stranded oligonucleotides 8a/b and 9a/b (minigene 2). As a result two pMad5-derived plasmids (pMad5-1, pMad5-2) were obtained coding for the recombinant proteins depicted in Fig 8. RAd were constructed by transfection of the Ad5E1-positive cell line 911 (58) with either plasmid pMad5-1 or pMad5-2 together with plasmid pJM17, which contains the sequence of the Ad5 mutant d1309 (59). 911 cells were co-transfected with 10 μg of linearized plasmid pMad5-1, respectively pMad5-2 and 10 μg of plasmid pJM17. The resulting rAd, which arose through homologous recombination between pMAd5 and pJM17, were 3 times plaque-purified, and subsequently propagated in 911 cells, purified by double cesium-chloride density centrifugation and extensively dialysed. The presence of revertants was routinely checked by infection of HEP-G2 cells. The viral stocks were stored in aliquots with 10% glycerol at -80°C and titered by plaque assay using 911 cells.

Transfection of COS-7 cells.

Transient transfection in COS-7 cells was performed as described elsewhere (60). In short, 100 ng of Plasmids encoding Ad5E1, HPV16 E7, murine p53, or the influenza-matrix protein together with 100 ng of a plasmid encoding H-2D$^b$, H-2Kb or HLA-A*0201 were transfected by the DEAE-dectran-chloroquine method into 1x10$^4$ COS-7 cells. The transfected COS cells were incubated in 100 µl Iscove's modified Dulbecco's medium containing 8% FCS for 48 h at 37°C, after which 1500-500 CTL in 25 µl Iscove's modified Dulbecco's medium containing 50 Cetus Units of recombinant Interleukin-2 (rIL-2, Cetus Corp., Emeryville, CA, USA) were added. After 24 h, the supernatant was collected and its tumor necrosis factor (TNF) content was determined by measuring its cytotoxic effect on WEHI-164 clone 13 cells as previously described (60).

Infection of MEC with rAd

B6 MEC were infected with rAd diluted in 1 ml Iscove's modified Dulbecco's medium containing 0.5% bovine serum albumine. After 30 minutes at room temperature Iscove's modified Dulbecco's medium containing 10% FCS was added. The multiplicity of infection (MOI) (for B6 MEC a moi of 50 was used) was chosen to give at least 80% of infected cell. This was determined by infection with Ad.RSVβ-Gal carrying the Escherichia coli LacZ gene, encoding β-galactosidase under control of the promotor from the rous sarcoma virus long terminal repeat, followed by X-gal staining 48 hours later.

Generation of CTL bulk cultures

5 x 10$^6$ spleen cells per well, derived from B6 mice taken 2 weeks or more after the intra-peritoneal immunization with 1 x 10$^8$ plaque forming units (PFU) of rAd or the replication-defective Ad5-mutant Ad5 ts 149 were co-cultured for 6 days with 10% irradiated (25GY) IFN-γ (10 units/ml) treated stimulator cells in 24-wells plates. Next, effector cells were harvested and dead cells were removed by density centrifugation on lympholyte M. These cells were used in a cell-mediated lymphocyte cytotoxicity assay.

Cell-mediated lymphocyte cytotoxicity.

Experimental procedures to measure cell-mediated cytotoxicity were performed in an Europium- (Eu$^{3+}$) release assay as described elsewhere (56). In short, varying numbers of effector cells were added to 10$^3$ Eur$^{3+}$-labeled target cells in 0.15 ml of culture medium in 96-well U-bottomed plates. After a 4 hour incubation at 37°C, supernatants were collected and mixed with Enhancer solution® (Wallac, Turku, Finland). Measurement of the samples took place in a 1234 Deifia® fluorometer (Wallac). The mean percentage specific lysis of triplicate wells was calculated as follows:

% Specific lysis = [(cpm experimental release - cpm spontaneous release)/(cpm maximum release - cpm spontaneous release)] x 100.

Peptides.

Peptides were generated by solid phase strategies on a multiple peptide synthesizer (Abimed AMS 422) as described previously (61).

Tumor cell challenge.

C57BL/6 mice were immunized intra-peritoneally with 1 x 10$^8$ plaque forming units (PFU) or rAd or the replication-defective Ad5-mutant Ad5 ts 149 in 0.25 ml PBS/BSA. Two weeks later the mice were sub-cutaneously challenged with 0.4 x 10$^6$ Ad5E1A + ras cells in 0.25 ml PBS. Tumor volumes were measured with a caliper. Animals were sacrificed when their tumors grew larger than 1000$^3$ mm to avoid unnecessary suffering.

**RESULTS**

Insertion of the coding sequences of several CTL epitopes into pMad5

Vaccination with recombinant viruses encoding intact oncoproteins is intrinsically hazardous, because it can lead to transformation of recombinant virus-infected cells. Therefore, we set out to assemble two minigenes encoding several different CTL epitopes, that were cloned behind the major-late promotor of the vector pMad5. Since we set out to study whether rAd expressing several CTL epitopes in a string-of-bead fashion can be used for vaccination purposes the CTL epitopes used for the construction of the minigene were selected on basis of the availability of CTL clones recognizing the CTL epitopes and/or tumor cells expressing the CTL epitopes. Based upon current knowledge of antigen processing and presentation the CTL epitopes were separated from each other by a spacer of three alanines. The incorporation of the proteolytic cleavage site of three alanines ensures that the encoded CTL eptipes are properly processed (62). The availability of CTL clones recognizing the minigene-encoded CTL epitope is important in order to determine whether the minigene is translated and whether the encoded CTL epitopes are presented in the context of the proper MHC class I-molecule. Likewise, the available murine tumor-models can be used as read-out in order to determine whether the constructed rAd are able, upon vaccination, to induce protective respectively therapeutic CTL mediated anticancer immunity.

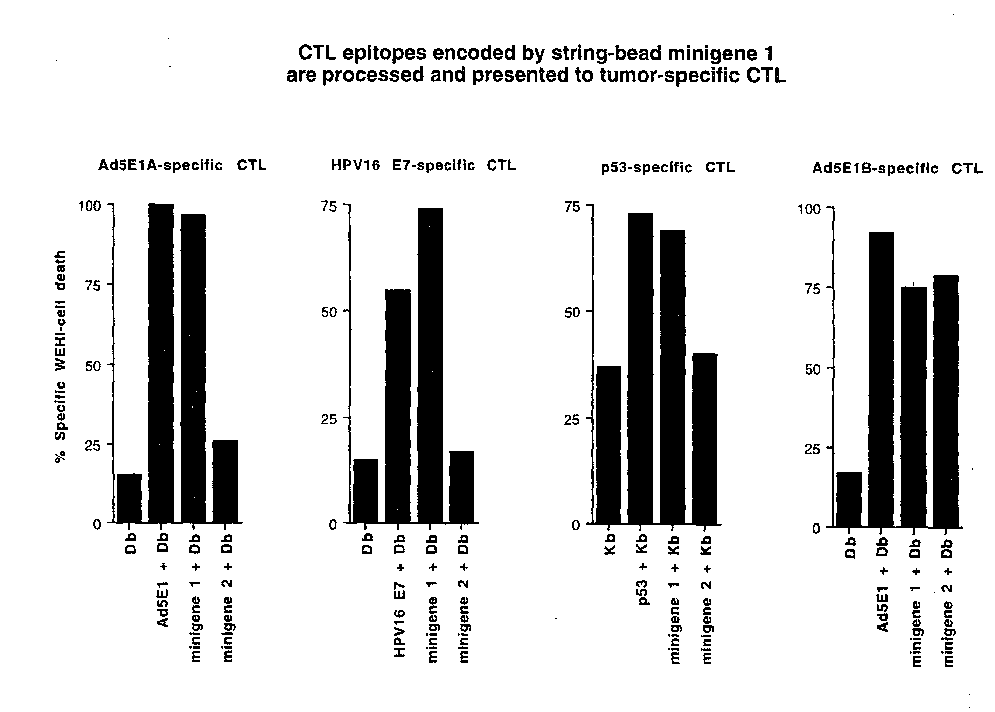

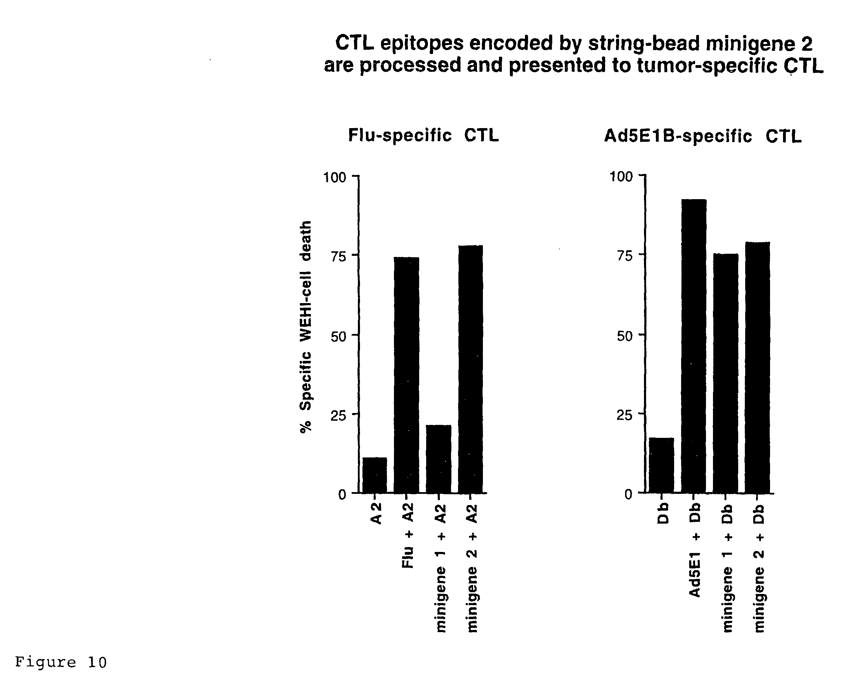

Based upon these considerations we generated two recombinant adenoviruses encoding two synthetic minigenes (Fig 8). The synthetic minigenes encoding the CTL epitopes depicted in figure 8 were cloned into plasmid pMad5 as described in the material and methods section. All CTL epitopes encoded by pMad5-1 and two of the four CTL epitopes encoded by pMad-2 were shown to be processed and presented to tumor-specific CTL as is shown in transient transfection experiments (Fig 9 and Fig 10). Processing and presentation of the HPV16 -derived HLA-A2-restricted CTL epitopes incorporated in pMad5-2 could not be tested, due to the fact that no CTL clones specific for these peptides are currently available. Nonetheless, our data indicate that these peptides are expressed and most likely processed, since the last (Ad5E1B-derived) CTL epitope in the construct is translated, processed and presented to Ad5E1B-specific CTL. We, therefore, conclude that all CTL epitopes encoded by pMad5-1 and pMad5-2 are translated, processed and presented to CTL clones.

Since introduction of the minigenes into cells leads to presentation of the desired CTL epitopes in the context of the appropriate MHC-restriction molecules, the plasmids pMad5-1 and pMad5-2 harbouring minigene 1 or 2 have been used to generate replication-defective rAd.

<u>The CTL epitopes encoded by the constructed rAd are processed and presented to tumor-specific CTL.</u>

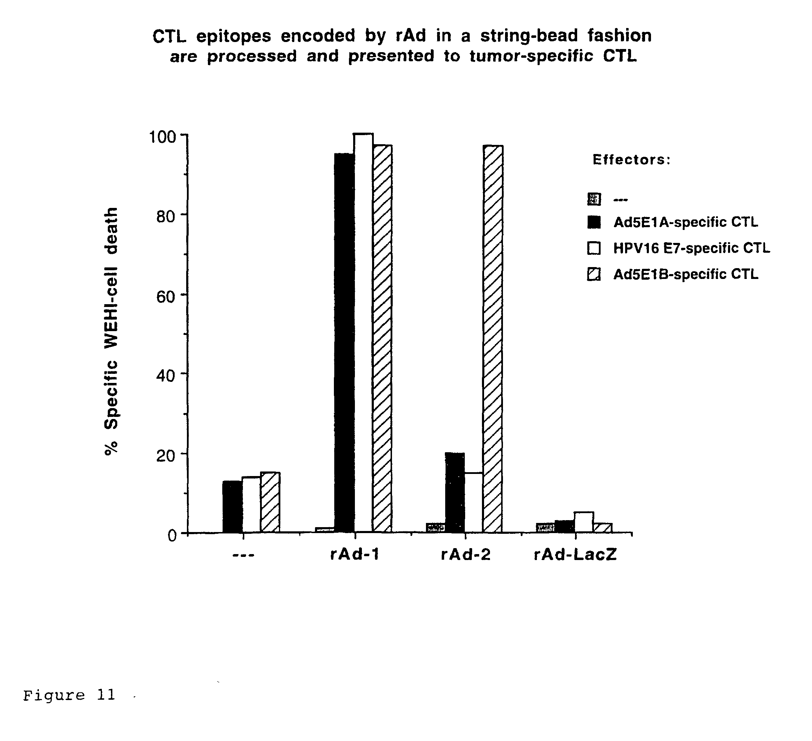

In order to analyse whether the generated rAd are able, upon infection, to activate tumor-specific CTL clones, B6 MEC have been infected with the constructed rAd. These rAd-infected MEC were used as stimulator cells in a T cell activation assay, using TNF-prouction as read-out. For reasons of convenience, we focussed in these experiments on the H-2$^{b}$-encoded, virus-derived CTL epitopes. Upon infection with the rAd encoding minigene 1 (rAd-1), both the Ad5E1A-, HPV16 E7-, and the Ad5E1B-derived CTL epitopes are presented to the appropriate CTL, since these CTL were activated when incubated with B6 MEC infected with this virus, but not when incubated with B6MEC infected with a control rAd (Fig 11). By infection of MEC derived from p53 knock-out mice we were able to show that also the p53-derived CTL epitope was efficiently processed and presented to p53-specific CTL (data not shown). Likewise, the rAd encoding minigene 2 (rAd-2) is able to deliver the Ad5E1B-derived CTL epitope, since infection of B5 MEC with this virus leads to activation of Ad5E1B-specific CTL (Fig 11). Thus, the constructed rAd are able to deliver all pre-selected CTL epitopes to tumor-specific CTL.

5. <u>Vaccination of B6 mice with rAd induces tumor-reactive CTL activity</u>.

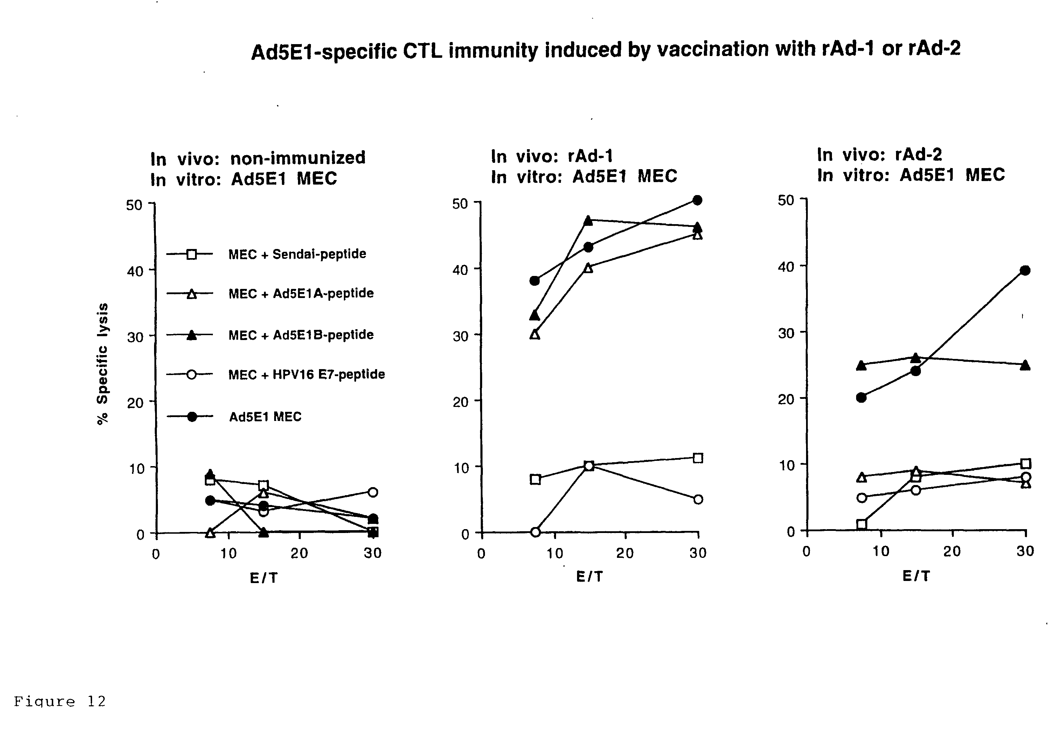

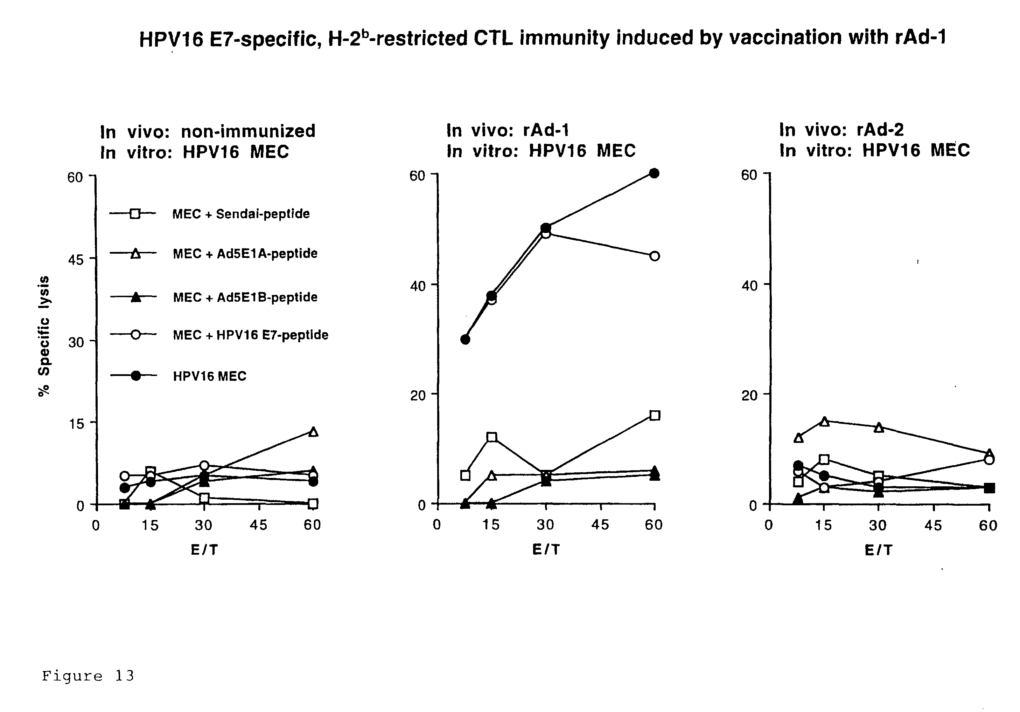

Since the rAd are able to deliver all three H-2-restricted viral CTL epitopes, we have analysed whether vaccination with these viruses induce CTL activity against these CTL epitopes. Indeed, bulk CTL cultures derived from B6 mice immunized with the rAd-1 display high CTL activity against the Ad5E1A-. HPVI6 E7-, and the Ad5E1B-encoded CTL epitopes (Fig. 12 and Fig. 13). Moreover, these CTL bulk cultures also lyse tumor cells harbouring the relevant CTL epitopes, showing that the induced CTL display a strong anti-tumor activity. Similarly, vaccination of B6 mice with rAd-2 induced Ad5E1B-specific CTL activity that cross-reacted on Ad5E1B-expressing tumor cells (Fig. 12). Taken together, these data show that rAd harbouring synthetic minigenes encoding several CTL epitopes in a string-bead fashion are able to induce, upon vaccination, strong tumor-specific CTL responses against the CTL epitopes of choice.

<u>Immunization with rAd-1 induces protective immunity against a challenge with Ad5E1A + ras transformed tumor cells</u>.

The data described above show that immunization wtih rAd-1 or rAd-2 induce strong tumor-reactive CTL activity against all tested CTL epitopes. To test whether mice. vaccinated with rAd are also protected against a lethal challenge with tumor cells, we challenged these mice with tumor cells transformed by the Ad5E1A-region and an activated ras oncogene (53). These tumor cells only express the Ad5E1A-encoded CTL epitope, and it is therefore anticipated that rAd-1 only, but not rAd-2, is able to induce protective immunity against this tumor upon vaccination.

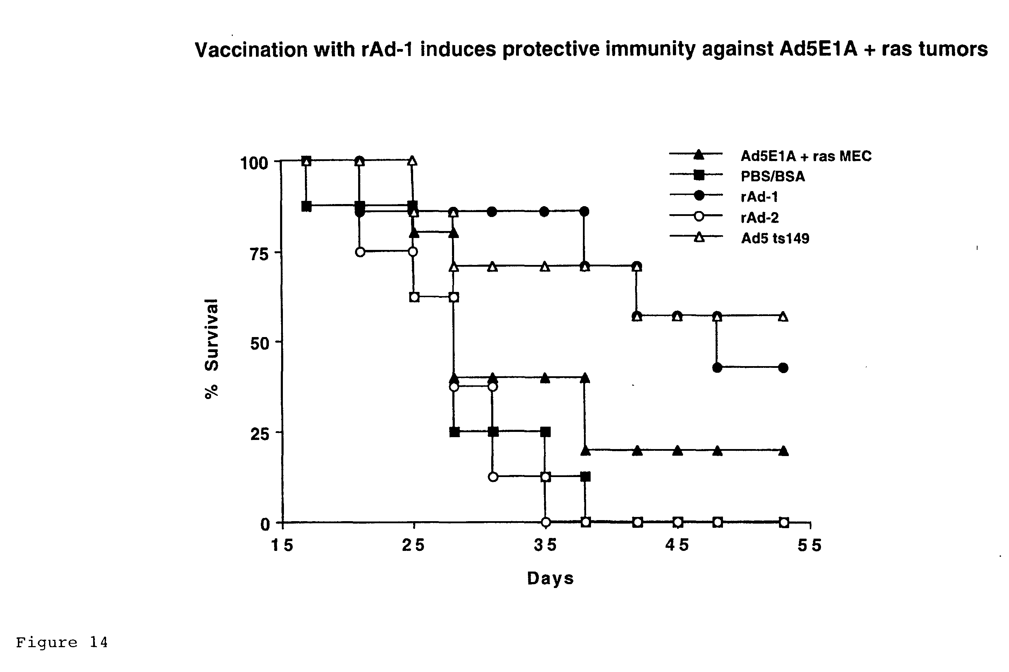

Indeed, mice immunized with rAd-1, but not mice immunized with rAd-2 or PBS/BSA only, were protected against the outgrowth of Ad5E1A + ras expressing tumor cells (Fig. 14). Moreover, the protection induced by vaccination with rAd-1 is better than that obtained by vaccination with irradiated tumor cells, showing that vaccination with rAd is superior compared to other vaccination regimes. Thus, vaccination with rAd, harbouring several CTL epitopes, linked with a proteolytic cleavage site, is a powerful way to induce protective immunity directed against pre-selected T cell epitopes of choice.

Comments

This example shows that rAd encoding defined CTL epitopes in a string-of-bead fashion, in which the CTL epitopes are linked to each other by sequences that ensure efficient processing and presentation of the CTL epitopes are very potent in inducing protective CTL responses against tumors. All CTL epitopes encoded by the rAd were processed and presented to tumor- and virus-specific CTL, illustrating that multiple CTL epitopes can be delivered to the host by a single vaccination, leading to strong and protective CTL responses. rAd are easy to manufacture, and do not cause side-effects when used for vaccination, in contrast to other carriers as vaccinia. Therefore, this method of vaccination is very effective and safe and is currently being used to deliver other CTL epitopes described in this invention.

**Example 5.**

Along the same way as described in Example 4, a vaccine will be prepared, in which the CTL epitopes are incorportated described in Tables X - XX. The vaccine is prepared with the following characteristics:

a. the vaccine contains several T cell epitopes linked to each other by a spacer
b. the spacers contain proteolytic cleavage sites
c. the T cell epitope containing construct is delivered by a recombinant adenovirus or is incorporated into the vaccine types described by point iii - vii on pages 2 and 3.

A vaccine for melanoma is prepared harbouring peptides mentioned in Table XI, a vaccine for colon carcinoma is prepared harbouzing peptides mentioned in Tables XII and XX, a vaccine for cervical carcinoma is prepared harbouring peptides mentioned in Tables XIII - XVI, a vaccine for HIV is prepared harbouring the peptides mentioned in Table XIX. When appropriate, peptide T cell epitopes other than the ones listed in Tables X - XX are incorporated into these multi-epitope vaccines. The T cell epitopes present in these vaccines are linked to each other by the following proteolytic cleavage sites (or part of these proteolytic cleavage sites):
AAA as described in (62)
QGW*FEG, WFE*GLF, FEG*LFN, FTT*LIS, TTL*IST, TLI*STI, FNK*SPW, EGL*FNK, TTL*IST, TLI*STI, FNR*SPW as described in (63)
VSG*LEQ, SII*NFE, INF*EKL, LTE*WTS, IIN*FEK, GLE*QLE, EQL*ESI,
NFE*KLT, QLE*SII, EKL*TEW, VVR*FDK, STR*TQI, TQI*NKV,
KVV*RFD, VVR*FDK, VRF*DKL, RFD*KLP, DKL*PGF, FGD*SIE,
VSG*LEQ, QLE*KVV, FDK*LTE, KLT*EWT as described in (64, 65)
LMY*DMY, SEK*RVV, KRV*WMS, DMY*PHF, TNL*GPS, LMY*DMY, as described in (66)
LYE*NKP as described in (67)
VNQ*HLC, SHL*VEA, LVE*ALY, EAL*YLV, LYL*VCG as described in (68)
VNQ*HLC, QHL*CGS, LVE*ALY, EAL*YLV, ALY*LVC, LYL*VCG,
YLV*CGE, LVC*GER, RGF*FYT, GFF*YTP, FFY*TPK, FYT*PKA,
YTP*KA, TPK*A as described in (69)
whereby * represent the site after which the proteasome complex cleaves.
RAd vaccines carrying multi-epitope constructs as described above are applied in the appropriate clinical setting as follows:
Dose:
between $10^5$ and $10^{11}$ pfu
diluent: isotonic solution; 100 - 1000 µl
Administration:
One to three times, at two-to four-week intervals
Possible sites:
Subcutaneous, intra-cutaneous, intra-peritoneal, intramuscular
Clinical evaluations:

Inhibition of tumor-growth, regression of existing tumors/metastases
Immunological evaluation:
Measurement of T cell responses against relevant and control peptide T cell epitopes before and after vaccination.

Legends to Figures Example 4

Fig. 8. Minigenes encoding several CTL epitopes, linked by a spacer of three alanines.

The first minigene (rAd-1) encodes an Ad5E1A$_{234-243-}$ encoded, H-2D$^b$-restricted CTL epitope (55), an HPV16E7$_{49-59-}$ encoded, H-2D$^b$-restricted CTL epitope (70), a p$^{53}$$_{158-166-}$ encoded, H-2K$^b$-restricted CTL epitope (unpublished results), an Ad5E1B$_{192-200}$-encoded, H-2D$^b$-restricted CTL epitope (56), and a Myc-Tag.

The second minigene (rAd-2) encodes an HPVI6 E7$_{86-93-}$ encoded, HLA-A*0201-restricted CTL epitope (71), an Flu-matrix$_{58-66}$, HLAA*0201-restricted CTL epitope (72), An HPVI6 E711-20-encoded, HLAA*0201-restricted CTL epitope (71), an Ad5E1B$_{192-200}$-encoded, H-2D$^b$restricted CTL epitope (56), and a Myc-Tag.

Fig. 9. Minigene I-encoded CTL epitopes are presented to tumor-specific CTL clones. pMad5-1 was transfected, together with a plasmid encoding the appropriate restriction element, into COS-7 cells. After 48 hours, the transfected COS-7 cells were tested for the expression of the CTL epitopes in their ability to cause TNF-release by the relevant CTL. The presence of TNF in the culture supernatant was measured by the cytotoxic effect on WEHI-164 clone 13 cells.

All relevant CTL were activated by COS-7 cells transfected with a plasmid encoding minigene 1 (but not an irrelevant control plasmid) together with a plasmid encoding the appropriate restriction molecule. Thus, minigene 1 is translated into protein and the encoded CTL epitopes are processed and presented in the context of the appropriate MHC-molecule to tumor-specific CTL.

Fig. 10. The Flu-derived and Ad5ElB-derived CTL epitopes are presented to Flu-, respectively, Ad5E1B-specific CTL by minigene 2. pMad5-2 was transfected, together with a plasmid encoding the appropriate restriction element, into COS-7 cells. After 48 hours, the transfected COS-7 cells were tested for the expression of the CTL epitopes in their ability to cause TNF-release by the relevant CTL. The presence of TNF in the culture supernatant was measured by the cytotoxic effect on WEHI-164 clone 13 cells. Relevant CTL were activated by COS-7 cells transfected with this plasmid (but not an irrelevant control plasmid) and a plasmid encoding the appropriate restriction molecule. Tbus, minigene 2 is translated into protein and encoded CTL epitopes are processed and presented in the context of the appropriate MHC-molecule to specific CTL.

Fig. 11. CTL epitopes encoded by rAdV are processed, and presented to tumorspecific CTL. B6 MEC were left uninfected, or were infected with rAd-1 harbouring minigene 1, rAd-2, harbouring minigene 2 or the galactosidase gene (RAdV-LAC-Z) at an multiplicity of infection of 50. Two days later these cells were used in a TNF-production assay as described above. B6 MEC infected with the rAd-1 harbouring Ad5E1A-, HPV16 E7-and Ad5E1B-derived H-2D$^b$-restricted CTL epitopes are able to activate CTL clones. specific for these CTL epitopes, whereas B6 MEC infected with the rAd-2 harbouring an Ad5E1B-derived CTL only activate Ad5E1B-specific CTL. The CTL are not activated upon incubation with uninfected MEC or MEC infected with a control rAd.

Fig. 12. Vaccination with rAdV leads to induction of tumor-reactive CTL activity against the Ad5E1-encoded CTL epitopes. B6 mice were left non-immunized, were immunized with rAd-1, harbouring minigene 1, or were immunized with rAd-2, harbouring minigene 2. Two weeks later the spleens of these animals were taken and restimulated with Ad5E1-transformed tumor cells in order to propagate Ad5E1A- and Ad5E1B-specific CTL. Lytic activity of bulk CTL cultures was tested 6 days later on Ad5E1 MEC, B6 MEC loaded with the the Sendai-virus encoded control CTL epitope FAPGNYPAL, or the Ad5E1A-encoded CTL epitope SGPSNTPPE1, or the Ad5E1B-encoded CTL epitope VNIRNCCYI, or the HPV16 E7-encoded CTL epitope RAHYNIVTF. Mice immunized with rAd-1 recognize the Ad5E1A- and Ad5E1B-encoded CTL epitopes as well as-tumor cells endogenously presenting the Ad5E1A- and the Ad5E1B-epitope. Mice immunized with rAd-2 recognize the Ad5E1B-epitope as well as tumor cells endogenously presenting the Ad5E1B-encoded CTL epitope, whereas non-immunized mice do not display reactivity against the target cells. % specific lysis at different effector to target cell ratio's is shown.

Fig. 13. Vaccination with rAdV leads to the induction tumor-reactive CTL activity directed against the HPV16 E7, H-2D$^b$-restricted CTL epitope. B6 mice were left non-immunized, were immunized with rAd-1, harbouring minigene 1, or were immunized with rAd-2, harbouring minigene 2. Two weeks later the spleens of these animals were taken and restimulated with HPV16-transformed tumor cells in order to propagate H-2Db, HPV16 E7-specific CTL. Lytic activity of bulk CTL cultures was tested 6 days later on HPV16 MEC, B6 MEC loaded with the the Sendai-virus encoded control CTL epitope FAPGNYPAL, or the Ad5E1A-encoded CTL epitope SGPSNTPPE1, or the Ad5E1B-encoded CTL epitope VNIRNCCYI, or the HPVI6 E7-encoded CTL epitope RAHYNIVTF. Mice immunized with rAd-1 recognize the HPV16 E7-encoded CTL epitopes as well as tumor cells endogenously presenting the HPV16 E7-epitope. Non-immunized mice and mice immunized with rAd-2 do not display reactivity against HPV16 E7-peptide positive target cells. % specific lysis at different effector to target cell ratio's is shown.

Fig. 14. Vaccination with rAd-1 induces protective immunity against a lethal challenge with Ad5E1A-expressing tumor cells. B6 mice were immunized intraperitoneally with rAd-1, rAd-2, the Ad5-mutant (Ad5E1A-positive) Ad5ts149, sub-cutaneously with 10 xOl irradiated Ad5E1A + ras transformed tumor cells, or were injected with PBS/BSA only. Two weeks later the mice received a subcutaneous challenge of 0.4 x $10^6$ Ad5E1A + ras cells. Mice immunized with rAd-1 and Ad5ts149 are protected against the outgrowth of Ad5E1A + ras cells, showing that immunization with rAd induces protective immunity against tumors.

**§5 References**

**1.** D'Amaro, J., Houbiers, J.G.A., Drijfhout, J.W.D., Brandt, R.M.P., Melief, C.J.M. Kast, W.M. A computer program for predicting possible cytotoxic T lymphocyte epitopes based on HLA class I peptide-binding motifs. Human Imm., *43*: 13-18, 1995.

**2.** Nijman, H.W., Houbiers, J.G.A., Vierboom, M.P.M., Van den burg, S.H., Drijfhout, J.W., D'Amaro, J., Kenemans, P., Melief, C.J.M. Kast, W.M. Identification of peptide sequences that potentially trigger HLA-A2.1-restricted cytotoxic T lymphocytes. Eur. J. Immunol., *23*: 1215-1219, 1993.

**3.** Van der Burg, S.H., Ras, E., Drijfhout, J.W.D., Benckhuijsen, W.E., Bremers, A.J.A., Melief, C.J.M. Kast, W.M. An HLA class I peptide binding assay based on competition for binding to class I molecules on intact human B cells: identification of conserved HIV-1 polymerase peptides binding to HLA-A*0301. Hum. Immunol., *44*: 189-198, 1995.

**4.** Ossendorp, F., Eggers, M., Neisig, A., Ruppert, T., Groettrup, M., Sijts, A., Mengedé, E., Kloetzel, P.-M., Neefjes, J., Koszinowski, U. Melief, C.J.M. A single residue exchange within a viral CTL epitope alters proteasome-mediated degradation resulting in lack of antigen presentation. Submitted for publication., 1996.

**5.** Neisig, A., Roelse, J., Sijts, E.J.A.M., Ossendorp, F., Feltkamp, M.C.W., Kast, W.M., Melief, C.J.M. Neefjes, J. J. Major differences in TAP-dependent translocation of MHC class I presentable peptides and the effect of flanking sequnces. J. Immunol., *154*: 1273-1279, 1995.

**6.** Kast, W.M., Brandt, R.M.P., Sidney, J., Drijfhout, J.W., Kubo, R.T., Grey, H.M., Melief, C.J.M. Sette, A. Role of HLA-A motifs in identification of potential CTL epitopes in human papillomavirus type 16 E6 and E7 proteins. J. Immunol., *152*: 3904-3912, 1994.

**7.** Garboczi, D.N., Hung, D.T. Wiley, D.C. HLA-A2-peptide complexes: refolding and christallization of molecules expressed in E.coli and complexed with single antigenic peptides. Proc.Natl.Acad.Sci.USA, *89*: 3429-3433, 1992.

**8.** Parker, K.C., Silver, M.L. Wiley, D.C. An HLA-A2/β2-microglobulin/peptide complex assembled from subunits e expressed separately in E.coli. Mol.Immunol., *29*: 371-378, 1992.

**9.** Feltkamp, M.C.W., Vierboom, M.P.M., Toes, R.E.M., Ossendorp, F., Ter Schegget, J., Melief, C.J.M. Kast, W. M. Competition inhibition of cytotoxic T lymphocyte (CTL) lysis, a ore sensitive method to identify candidate CTL epitopes that antibody-detected MHC class I stabilization. Immunol. Letters, *in press*: 1995.

**10.** Sette, A., Vitiello, A., Reherman, B., Fowler, P., Nayersina, R., Kast, W.M., Melief, C.J.M., Oseroff, C., Yuan, L., Ruppert, J., Sidney, J., Del Guerco, M.F., Southwood, S., Kubo, R.T., Chesnut, R.W., Grey, H.M. F.V., C. The relationship between class I binding affinity and immunogenicity of potential cytotoxic T cell epitopes. J. Immunol., *153*: 5586-5592, 1994.

**11.** Castelli, C., Storkus, W.J. Mauerer, B.M. J.Exp.Med., *181*: 363-368, 1995.

**12.** Cox, A.L., Skipper, J., Chen, Y., Henderson, R.A., Darrow, T.L., Shabanowitz, J., Engelhard, V.H., Hunt, D.F. Slingluff, C.L. Identification of a peptide recognized by five melanoma-specific cytotoxic T cell lines. Science, *264*: 716-719, 1994.

**13.** Nijman, H.W., Houbiers, J.G.A., Van der Burg, S.H., Vierboom, M.P.M., Kenemans, P., Kast, W.M. Melief, C. J.M. Characterization of cytotoxic T lymphocyte epitopes of a self-protein, p53, and a non-self-protein, influenza matrix: relationship between MHC peptide binding affinity and immune responsiveness to peptides. J. Immunother., *14*: 121-126, 1993.

**14.** Sette, A., Sidney, J., Del Guercio, M.F., Southwood, S., Ruppert, J., Grey, H.M. Kubo, R.T. Peptide binding to the most frequent HLA-A class I alleles measured by quantative molecular binding assays. Mol. Immunol., *31*: 813-822, 1994.

**15.** Myers, G., Wain-Hobson, S., Korber, B., Smith, R.F. Pavlakis, G.N. in , Los Alamos National Laboratory, Los Alamos, 1993,

**16.** Kubo, R.T., Sette, A., Grey, H.M., Apella, E., Sakaguchi, K., Zhu, N.Z., Arnott, D., N., S., Shabanowith, J., Michel, H., Bodnar, W.M., Davis, T.A. Hunt, D.F. Definition of specific peptide motifs for four major HLA-A alleles. J.Immunol., *152*: 3913-3926, 1994.

**17.** Engelhard, V.H. Structure of peptides associated with MHC class I molecules. Curr. Opin. Immunol., *6*: 13-23, 1994.

**18.** Bremers, A.J.A., Van der Burg, S.H., Kuppen, P.J.K., Kast, W.M., Van de Velde, C.J.H. Melief, C.J.M. The use of Eppstein Barr virus-transformed B lymphocyte cell lines in a peptide-reconstitution assay: Identification of CEA-related HLA-A*0301-restricted potential cytotoxic T lymphocyte epitopes. J.Immunother., *18*: 77-85, 1995.

**19.** Storkus, W.J., Zeh III, H.J., Salter, H.D. Lotze, M.T. Identification of T cell epitopes: Rapid isolation of class I presented peptides from viable cells by mild acid elution. J. Immunother., *14*: 94-105, 1993.

**20.** Suguwara, S., Abo, T. Kumagai, K. A simple method to eliminate the antigenicity of surface class I MHC molecules from the membrane of viable cells by acid treatment at pH 3. J. Immunol. Methods, *100*: 83-90, 1987.

**21.** Ljunggren, H.-G., Stam , N., J,, Öhlen, C., Neefjes, J.J., Höglund, P., Meemels, M.-T., Bastin, J., Schumacher, T.N.M., Townsend, A., Kärre, K. Ploegh, H.L. Empty MHC class I molecules come out in the cold. Nature, *346*: 476-480, 1990.

**22.** Cerundolo, V., Elliott, T., Elvin, J., Bastin, J., Rammensee, H.-G. Townsend, A. The binding affinity and dissociation of peptides for class I major histocompatibility complex molecules. Eur. J. Immunol., *21*: 2069-2075, 1991.

**23.** Neefjes, J.J., Diers, J. Ploegh, H.L. The effect of anchor residue modifications on the stability of major histocompatibility complex class 1-peptide interactions. Eur. J. Immunol., *23*: 840-845, 1993.

**24.** Ojcius, D.M., Abastado, J.-P., Casrouge, A., Mottez, E., Cabanie, L. Kourilsky, P. Dissociation of the peptide-MHC class I complex limits the binding rate of exogenous peptide. J. Immunol., *151*: 6020-6026, 1993.

**25.** Vitiello, A., Marchesini, D., Fruze, J., Sherman, L.A. Chesnut, R.W. Analysis of the HLA-restricted influenza-specific CTL response in transgenic mice carrying a chimeric human-mouse class I MHC. J.Exp.Med., *173*: 1007-1018, 1991.

**26.** Milich, D.R.J.L., Hughes, L.J., McLachlan, A., Thornton, G.B. Moriarty, A. Hepatitus B synthetic immunogen comprised of nucleocapsid T cell sites and envelop B protein. Proc.Natl.Acad.Sci.USA, *85*: 1610-1615, 1988.

**27.** Van der Burg, S.H., Klein, M.R., Van de Velde, C.J.H., Kast, W.M., Miedema, F. Melief, C.J.M. Induction of primary human cytotoxic T lymphocyte response against a novel conserved epitope in a functonal sequence of HIV-1 reverse transcriptase. AIDS, *9*: 121-127, 1995.

**28.** Parham, P. Brodsky, F.M. Partial purification and some properties of BB7.2. A cytotoxic monoclonal antibody with specificity for HLA-A2 and a variant of HLA-A28. Hum. Immunol., *3*: 277-286, 1981.

**29.** Ressing, M.E., Sette, A., Brandt, R.M.P., Ruppert, J., Wentworth, P.A., Hartman, M., Oseroff, C., Grey, H.M., Melief, C.J.M. Kast, W.M. Human CTL epitopes encoded by human papillomavirus type 16 E6 and E7 identified through in vivo and in vitro immunogenicity studies of HLA-A*0201-binding peptides. J. Immunol., *154*: 5934-5943, 1995.

**30.** Cerny, A., McHutchinson, J.G., Pasquinelli, C., Brown, M.E., Brothers, M.A., Grabscheid, B., Fowler, P., Houghton, M. Chisari, F.V. Cytotoxic T lymphocyte response to hepatitis B virus-derived peptides containing the HLA-A2.1 binding motif. J.Clin.Invest., *95*: 1995.

31. Nayersina, R., Fowler, P., Guihot, G., Missale, G., Cerny, A., Schlicht, H.J., Vitiello, A., Chesnut, R., Person, J.L., Redeker, A.G. Chisari, F.V. HLA-A2 resricted cytotoxic T lymphocyte responses to multiple hepatitus B surface antigen epitopes during hepatitis B virus infection. J. Immunol., *150*: 4659-4666, 1993.

**32.** Lee, S.P., Thomas, W.A., Murray, R.J., Khanim, F., Kaur, S., Toung, L.S., Rowe, M., Kurilla, M. Rickinson, A. B. HLA A2.1-restricted cytotoxic T cells recognizing a range of Eppstein Barr virus isolates through a defined epitope in latent membrane protein. J.Virol., *67*: 7428-7439, 1993.

**33.** Utz, U., Koenig, S., Coligan, J.E. Biddison, W.E. Presentation of three different viral peptides, HTLV-1 TAX, HCMV gB and influenza virus M1, is determined by common structural features of the HLA-A2.1 molecule. J. Immunol., *149*: 214-223, 1992.

**34.** Tarpey, I., Stacey, S., Hickling, J., Birley, H.D.L., Renton, A., McIndoe, A. Davies, D.H. Human cytotoxic T lymphocytes stimulated by endogeneously processed HPV11 E7 recognize a peptide containing a HLA-A2 (HLA-A*0201) motif. Immunology, *81*: 222-227, 1994.

**35.** Robbins, P.A., Lettice, L.A., Rota, P., Santos-Aguado, J., Rothbard, J., McMichael, A.J. Strominger, J.L. Comparison between two peptide epitopes presented to cytotoxic T lymphocytes by HLA-A2. Evidence for discrete locations within HLA-A2. J.Immunol., *143*: 4098-4112, 1989.

**36.** Morrison, J., Elvin, J., Latron, F., Gotch, F., Moots, R., Strominger, J.L. McMichael, A. Identification of the nonamer peptide from Influenza A matrix protein and the role of pockets of HLA-A2 in its recognition by cytotoxic T lymphocytes. Eur. J. Immunol., *22*: 903-..., 1992.

**37.** Johnson, R.P., Trocha, A., Yang, L., Mazzara, G.P., Panicali, D.L., Buchanan, T.M. Walker, B.D. HIV-1 gag-specific cytotoxic T lymphocytes recognize multiple highly conserved epitopes. J.Immunol., *147*: 1512-1524, 1991.

**38.** Tsomides, T.J., Walker, B.D. Eisen, H.N. An optimal viral peptide recognized by CD8+ T cells binds very tightly to the restricting class I MHC protein on intact cells but not to the purified class I protein. Proc. Natl. Acad. Sci. USA, *88*: 11276-, 1993.

**39.** Kawakami, Y., Eliyahu, S., Delgado, C.H., Robbins, P.F., Sagaguchi, K., Apella, E., Yanelli, J.R., Adema, G.

J.; Miki, T. Rosenberg, S.A. Identification of a human melanoma antigen recognized by tumor-infiltrating lymphocytes associated with in vivo tumor rejection. Proc.Natl.Acad.Sci.USA, *91*: 6458-6464, 1994.

**40.** Wölfel, T., Van Pel, A., Brichard, V., Schneider, J., Seliger, B., Meyer-Zum Buschenfelde, K.H. Boon, T. Two tyrosinase nonapeptides recognized on HLA-A2 melanomas by autologous cytolytic T lymphocytes. Eur.J.Immunol., *24*: 759-769, 1994.

**41.** De Kruijf, J., Boel, E. Logtenberg, T. Selection and application of human single chain Fv antibody fragments from a semi-synthetic phage antibody display library with designed CDR3 region. J. Mol. Biol., *248*: 97-105, 1995.

**42.** Visseren, M.C.W., Van Elsas, A., Van der Voort, E.I.H., Ressing, M.E., Kast, W.M., Schrier, P.I. Melief, C.J.M. CTL specific for the tyrosinase autoantigen can be induced from healthy donor blood to lyse melanoma cells. J. Immunol., *154:* 3991-3998, 1995.

**43.** Olsen, A.C., Fossum, B., Kirkin, A.F., Zeuthen, J. Gaudernack, G. Scand.J.Immunol., *41*: 357-369, 1995.

**44.** Kawakami, Y., Eliyahu, S., Sakaguchi, K., Robbins, P.F., Rivoltini, L., Yanelli, J.R., Apella, E. Rosenberg, S.A. J.Exp.Med., *180*: 347-358, 1994.

**45.** Bednarek, M.A., Sauma, S.Y., Gammon, M.C., Porter, G., Tamhankar, S., Williamson, A.R. Zweerink, H.J. The minimum peptide epitope from the influenza virus matrix protein. Extra and intra-cellular loading of HLA-A2. J. Immunol., *147*: 4047-4053, 1991.

**46.** Kawakami, Y., Eliyahu, S., Delgado, C.H., Robbins, P.F., Rivoltini, L., Topalian, S.L., Miki, T. Rosenberg, S.A. Cloning of the gene coding for a shared human melanoma antigen recognized by autologous T cells infiltrating into tumor. Proc. Natl. Acad. Sci. USA, *91*: 3515-3519, 1994.

**47.** Cox, A.L., Skipper, J., Chen, Y., Henderson, R.A., Darrow, T.L., Schabanowith, J., Engelhard, V.H., Hunt, D.F. Slingluff, C.L. Science, *264*: 716-722, 1993. References

**48.** Whitton, J.L., Sheng, N., Oldstone, M.B.A. McKee, T.A. A "string-of-beads" vaccine, comprising linked minigenes confers protection from lethal-dose virus challenge. J. Virol., *67*: 348-352, 1993.

**49.** Thomson, S.A., Elliott, S.L., Sherritt, M.A., Sproat, K.W., Coupar, B.E.H., Scalzo, A.A., Forbes, C.A., Ladhams, A.M., Mo, X.Y., Tripp, R.A., Doherty, P.C., Moss, D.J. Suhrbier, A. Recombinant polyepitope vaccines for the delivery of multiple cytotoxic T cell epitopes. J. Immunol., *157*: *822*-826, 1996.

**50.** Juillard, V., Villefroy, P., Godfrin, D., Pavirani, A., Venet, A. Guillet, J.-G. Long-term humoral and cellular immunity induced by a single immunization with replication-defective adenovirus recombinant vector. Eur. J. Immunol., *25*: 3467-3473, 1995.

**51.** Chen, P.W., Wang, M., Bronte, V., Zhai, Y., Rosenberg, S.A. Restifo, N.P. Therapeutic antitumor response after immunization with a recombinant adenovirus encoding a model tumor-associated antigen. J. Immunol., *156*: 224-231, 1996.

**52.** Zhai, Y., Yang, J.C., Kawakami, Y., Spiess, P., Wadsworth, S.C., Cardoza, L.M., Couture, L.A., Smith, A.E. Rosenberg, S.A. Antigen-specific tumor vaccines. Development and characterization of recombinant adenoviruses encoding MARTI and gp100 for cancer therapy. J. Immunol., *156*: 700-710, 1996.

**53.** Toes, R.E.M., Offringa, R., Blom, R.J.J., Melief, C.J.M. Kast, W.M. Peptide vaccination can lead to enhanced tumor growth through specific T-cell tolerance induction. Proc. Natl Acad. Sci. USA, *93*: 7855-7860, 1996.

**54.** Toes, R.E.M., Blom, R.J.J., Offringa, R., Kast, W.M. Melief, C.J.M. Functional deletion of tumor-specific cytotoxic T lymphocytes induced by peptide immunization can lead to the inability to reject tumors. J. Immunol., *156*: 3911-3918, 1996.

**55.** Kast, W.M., Offringa, R., Peters, P.J., Voordouw, A.C., Meloen, R.H., Van der Eb, A.J. Melief, C.J.M. Eradication of adenovirus E1-induced tumors by Ela-specific cytotoxic T lymphocytes. Cell, *59*: 603-615, 1989.

**56.** Toes, R.E.M., Offringa, R., Blom, H.J.J., Brandt, R.M.P., Van der Eb, A.J., Melief, C.J.M. Kast, W.M. An adenovirus type 5 early region IB-encoded CTL epitope-mediating tumor eradication by CTL clones is down-modulated by an activated ras oncogene. J. Immunol., *154*: 3396-3405, 1995.

**57.** Feltkwnp, M.C.W., Vreugdenhil, G.R., Vierboom, M.P.M., Ras, E., Van der Burg, S.H., Ter Schegget, J., Mehef, C.J.M. Kast, W.M. CTL raised against a subdominant epitope offered as a synthetic peptide eradicate human papillomavirus type 16-induced tumors. Eur.J. Immunol., *25*: 2638-2641, 1995.

**58.** Fallaux, F.J., Kranenburg, O., Cramer, S.J., Houweling, A., Van Ormondt, H., Hoeben, R.C. Van der Eb, A.J. Characterization of 911: a new helper cell line for the titration and propagation of early region 1-deleted adenoviral vectors. Hum. Gene Tber., *7*: 215-222, 1996.

**59.** Mc Grory, W.J., Bautista, D.S. Graham, F.L. A simple technique for the rescue of the early region I mutations into infectious human adenovirus type 5. Virology, *163*: 614-617, 1988.

**60.** Traversari, C., Van der bruggen, P., Van den Eynde, B., Hainaut, P., Lemoine, C., Ohta, N., Old, L. Boon, T. Transfection and expression of a gene coding for a human melanoma antigen recognized by autologous CTL. Immunogenetics, *35*: 145-152., 1992.

**61.** Gausepohl, H., Kraft, M., Boulin, C. Frank, R.W. in (eds. Rivier, J.E. & Marshall, G.R.), *11th American peptide symposium*, ESCOM, Leiden, 1990, 1003-1004.

**62.** Del Val, M., Schlicht, H.-J., Ruppert, T., Reddehase, M.J. Koszinowski, U. Efficient processing of an antigenic sequence for presentation by MHC class I molecules depends on its neigboring residues in the protein. Cell, *66*: 1145-1153, 1991.

**63.** Ossendorp, F., Eggers, M., Neisig, A., Ruppert, T., Groettrup, M., Sijts, A., Mengedé, E., Kloetzel, P.-M., Neefjes, J., Koszinowski, U. Melief, C.J.M. A single residue exchange within a viral CTL epitope alters proteasome-mediated degradation resulting in lack of antigen presentation. Immunity, 5:115-124, 1996.

**64.** Niedermann, G., Butz, S., Ihlenfeld, H.G., Grimm, R., Lucchiari, Hoschützky, H., Jung, G., Maier, B. Eichmann, K. Contribution of proteasome-mediated proteolysis to the hierarchy of epitopes presented by major histocompatibility complex class I molecules. Immunity, *2*: 289-299, 1995.

**65.** Dick, L.R., Aldrich, C., Jameson, S.C., Moomaw, C.R., Pramanik, B.C., Doyle, C.K., de Martino, G.N., Bevan, M.J., Forman, J.M. Slaughter, C.A. Proteolytic processing of ovalbumin and β-galactosidase by the proteasome to yield antigenic peptides. J. Immunol., *152*: 3884-3894, 1994.

**66.** Boes, B., Hengel, H., Ruppert, T., Multhaup, G., Koszinowski, U.H. Kloetzel, P.-M. Interferon stimulation modulates the proteolytic activity and cleavage site preference of 20 S mouse proteasomes. J. Exp. Med., *179*: 901-909, 1994.

**67.** Cardozo, C., Vinitzky, A., Hidalgo, M.C., Michaud, C. Orlowski, M. A 3,4-dichloroisocoumarin-restitant component of multicatalytic proteinase complex. Biochemistry, *31*: 7373-7380, 1992.

**68.** Dick, L.R., Moonaw, C.R., De Martino, G.N. Slaughter, C.A. Degradation of oxidized insulin B chain by the multiproteinase complex macropain (proteasome). Biochemistry, *30:* 2725-2734, 1991.

**69.** Ehring, B., Meyer, T. H., Eckerskom, C., Lottspeich, F. and Tampé, R. Effects of MHC-encoded subunits on the peptidase and proteolytic activities of human 20 Sproteasomes: Cleavage of proteins and antigenic peptides. Eur. J. Biochemistry *235*: 404-415, 1996.

**70.** Feltkamp, M.C.W., Smits, H.L., Vierboom, M.P.M., Minnaar, R.P., De Jongh, B.M., Drijfhout, J.W., Ter Schegget, J., Melief, C.J.M. Kast, W.M. Vaccination with a cytotoxic T lymphocyte epitope-containing peptide protects against a tumor induced by human Papillomavirus type 16-transformed cells. Eur. J. Immunol., *23*: 2242-2249., 1993.

**71.** Ressing, M.E., Sette, A., Brandt, R.M.P., Ruppert, J., Wentworth, P.A., Hartman, M., Oseroff, C., Grey, H.M., Melief, C.J.M. Kast, W.M. Human CTL epitopes encoded by human papillomavirus type 16 E6 and E7 identified through in vivo and in vitro immunogenicity studies of HLA-A*0201 -binding peptides. J. Immunol., *154*: 5934-5943, 1995.

**72.** Bednarek, M.A., Sauma, S.Y., Gammon, M.C., Porter, G., Tamhankar, S., Williamson, A.R. Zweerink, H.J. The minimum peptide epitope from the influenza virus matrix protein. Extra and intra-cellular loading of HLA-A2. J. Immunol., *147*: 4047-4053, 1991.

**73.** Levrero M., Barban, V., Tiollais, P., Pefficaudet, M. Defective and non-defective adenovirus vectors for expressing foreign genes in vitro and in vivo. Gene *101*: 195-202, 1991

**74.** Evan, G.I., Lewis, G.K., Ramsay, G. Bishop, M. Isolation of monoclonal antibodies specific for human c-Myc proto-oncogene product. Mol.Cell.Biol.*5*. 3610-3616, 1985.

**75.** Nelson, C.A., Petzold, S.J., Unanue, E.R. Peptides determine the lifespan of MHC class II molecules in the antigen-presenting cell. Nature 371: 250-252, 1994

**76.** Parker, K.C., Bednarek, M.A., Hull, L.K., Utz, U., Cunningham, B., Zweerink, H.J., Biddison, W.E., Coligan, J. E. Sequence motifs important for peptide binding to the human MHC class I molecule, HLA-A2.

TABLE 1 HPV 16 E6 and E7 peptides tested for binding in competition assay at different temperatures for different incubation times.

| Sequence[a] | HPV16 | $IC_{50}$[b] (nM) | Presence[c] of motif | 4°C 3 hr $IC_{50}$[d] (μM) | 4°C 24 hr $IC_{50}$ (μM) | 26°C 3 hr $IC_{50}$ (μM) | 26°C 24 hr $IC_{50}$ (μM) |
|---|---|---|---|---|---|---|---|
| HLA-A*0201 | | | | | | | |
| TLGIVCPI | E7 | 7 | + | 3.9 | 0.7 | 6.5 | 3.1 |
| LLHGTLGIV | E7 | 8 / 208 | + | 20 | 5 | 10 | 6.9 |
| YMLDLQPETT | E7 | 46 / 33 | + | 2.9 | 0.7 | 11 | 7.3 |
| TLGIVCPIC | E7 | 153 | - | >25 | 15.7 | 29 | 10 |
| KLPQLCTEL | E6 | 328 / 5556 | + | >25 | 8.3 | 36 | 34.5 |
| AMFQDPQER | E6 | 1818 / >25000 | - | >25 | 17.5 | >50 | >50 |
| LQTTIHDII | E6 | 3157 / >25000 | - | >25 | 20.3 | >50 | >50 |
| FLPSDCFPSV[e] | | - | + | 0.8 | 0.4 | 1 | 1.1 |
| HLA-A*0301 | | | | | | | |
| IVYRDGNPY | E6 | 10 / 660 | + | 2.1 | 0.8 | 2.5 | 2.5 |
| HLDKKQRFH | E6 | 68 | + | 1.9 | 1.7 | 9 | 17 |
| AMFQDPQER | E6 | 290 | + | 7.6 | 3.3 | 12.5 | 15 |
| TTLEQQYNK | E6 | 384 | + | 16.5 | 4.2 | 41 | 38 |
| IVCPICSQK | E7 | 1111 | + | 4.1 | 3.9 | 24 | 24 |
| AHSAARSSR | E6 | 4285 / 6600 | + | 4.3 | 4 | 6 | 7 |
| QQLLRREVY | E6 | 5000 / > 6600 | - | 17 | >25 | >50 | >50 |
| KVFPCALINK[e] | | - / 660 | + | 0.5 | 0.7 | 3 | 4 |

[a] amino-acid sequence of the HPV peptides

[b] binding capacity ($IC_{50}$) to the given HLA class I molecule as tested in the molecular binding assay [28]. For some peptides two $IC_{50}$ values are given: HLA-A*0201, the value at the right of the backslash was reported in a later publication [29]; HLA-A*0301, the value at the right side was determined using a molecular binding assay which employs the same FL-labeled reference peptide as used in the cellular binding assay [Drijfhout, manuscript in preparation].

[c] the presence of the HLA-A*0201 or HLA-A*0301 binding motif in the peptide

[d] binding capacity of the peptides is shown as the concentration of peptide needed to inhibit binding of the FL-labeled peptide to 50% ($IC_{50}$ in μM)

[e] the non-labeled reference peptides, the 'ash means that their $IC_{50}$ in the molecular binding assay is not known.

TABLE II

| Binding capacity of known processed and presented peptides | | | | |
|---|---|---|---|---|
| Sequence[a] | $IC_{50}$[b] (nM) | $IC_{50}$[c] (μM) | Origin | Reference |
| **HLA-A*0201** | | | | |
| FLPSDFFPSV | 2.8 | 0.4 | hepatitis B nucleocapsid | 30 |
| GILGFVFTL | 6 | 0.4 | influenza A matrix | 31 |
| ILKEPVHGV | 242 | 1.7 | HIV.1 RT | 32 |
| SLYNTVATL | | 1.3 | HIV-1 gag | 33 |
| YMNGTMSQV | - | 1.7 | tyrosinase | 34 |
| **HLA-A*0301** | | | | |
| QVPLRPMTYK | 11 | 0.5 | HIV-1 nef | 35 |
| KLFNIMVTY | · | 15 | unknown | 36 |
| KLHKORAKS | · | 12 | unknown | 36 |

[a] amino-acid sequence of the peptides
[b] binding capacity ($IC_{50}$ in nM) to the given HLA class I molecule as tested in the molecular binding assay [29]
[c] binding capacity of the peptides in the present study is shown as the concentration of peptide needed to inhibit binding of the FL-labeled peptide to 50% ($IC_{50}$ in μM)

TABLE III

| Binding of conserved HIV-1 pol sequences compliant with the HLA-A*0301 binding motif | | |
|---|---|---|
| Sequence[a] | Position[b] | $IC_{50}$ (μM)[c] |
| PISPIETVPVK | 160 - 170 | >100 |
| PIETVPVKLK | 163 - 172 | >100 |
| PIETVPVK | 163 - 170 | >100 |
| PLTEEKIK | 184 - 191 | >100 |
| AIKKKDSTK | 221 - 229 | 1.0 - 3.0 * |
| GIPHPAGLK | 252 - 260 | 0.3 - 0.5 * |
| SVTVLDVGDAY | 264 - 274 | >100 |
| TVLDVGDAY | 266 - 274 | >100 |
| VLDVGDAY | 267 - 274 | >100 |
| NVLPQGWK | 306 - 313 | 30.0 - 40.0 |
| WMGYELHPDK | 388 - 397 | 14.0 - 20.0 |
| ELELAENR | 459 - 466 | >100 |
| ELAENREILK | 461 - 470 | 14.0 - 20.0 |
| QLDCTHLEGK | 781 - 790 | 8.5 - 10.0 |
| AVHVASGY | 795 - 802 | 21.5 - 25.0 |
| QVRDQAEHLK | 883 - 892 | 2.9 - 3.0 * |
| AVFIHNFKR | 898 - 906 | 0.3 - 0.5 * |
| GIGGYSAGER | 909 - 918 | 6.5 - 10.0 |
| KIQNFRVYY | 938 - 946 | 1.8 - 2.5 * |
| KIQNFRVY | 938 - 945 | 70.0 - 90.0 |

[a] The amino-acid sequence of conserved peptides derived from HIV-1
[b] position of first and last amino-acid in HIV-1 polymerase derived from strain JR-CSF
[c] Peptides were tested in the competition assay at 4°C with an incubation time of 24 hours.

The binding capacity of the peptides is shown as the range of the concentration of peptide needed to inhibit binding of the FL-labeled peptide to 50% ($IC_{50}$ in μM). The peptides that are marked with a asterisk (*) are considered to be potential CTL epitopes.

Table IV

Comparison of the immunogenicity of Hepatitis B virus (HBV) or Human Papilloma virus type 16 (HPV16) derived peptides to the dissociation rate.

| Peptide | amino-acid position | Sequence | Affinity | | Immunogenicity | Stability (DT50%) | |
|---|---|---|---|---|---|---|---|
| | | | IC50 (nM) | IC50 (µM) | | | |
| *a* | | | | *b* | *c* | | *d* |
| HBV Pol | 635- 643 | GLYSSTVPV | 33 | 4.5 | + | > 4 | hr |
| HBV Pol | 1076-1084 | HLYSHPIIL | 38 | 8.0 | + | > 4 | hr |
| HBV Pol | 1344-1352 | WILRGTSFV | 278 | 11.0 | - | 1 | hr |
| HBV Pol | 996-1004 | NLSWLSLDV | 385 | 6.0 | + | 3 | hr |
| HBV Pol | 992-1000 | LLSSNLSWL | 1087 | 19.5 | - | 1 | hr |
| HBV Pol | 985- 993 | NLQSLTNLL | 2000 | 22.0 | - | NS | |
| HBV Pol | 43- 51 | HLLVGSSGL | 2778 | 24.0 | - | < 1 | hr |
| HBV Pol | 28- 36 | LLDDEAGPL | >25000 | 69.0 | - | NS | |
| HBV Pol | 594- 602 | PLEEELPRL | >25000 | >100 | - | NS | |
| HPV16 E7 | 86- 93 | TLGIVCPI | 7 | 0.7 | + | > 4 | hr |
| HPV16 E7 | 11- 20 | YMLDLQPETT | 46 | 0.7 | + | > 4 | hr |
| HPV16 E6 | 52- 60 | FAFRDLCIV | 130 | 9.0 | - | 2 | hr |
| HPV16 E7 | 7- 15 | TLHEYMLDL | 188 | 5.0 | - | 2 | hr |
| HPV16 E7 | 82- 90 | LLMGTLGIV | 208 | 5.0 | + | > 4 | hr |
| HPV16 E6 | 18- 26 | KLPQLCTEL | 328 | 8.5 | - | 2 | hr |
| HPV16 E6 | 7 - 15 | AMFQDPQER | 1818 | 17.5 | - | NS | |
| HPV16 E6 | 26- 34 | LQTTIHDII | 3157 | 20.5 | - | NS | |

*a* Peptide origin, position of first and last amino-acid and amino-acid sequence and binding affinity as described previously (10,24)

*b* Affinity was measured as described recently (17). IC$_{50}$ represents the amount of peptide required for 50% inhibition of binding of the fluorescein-labeled reference peptide to HLA-A*0201

*c* Immunogenicity of the peptide was determined by injection of peptide doses of 10- to 100-fold in excess of what is required to elicit optimal CTL responses emulsified in IFA together with an equimolar amount of I-A° T-helper epitope (10, 11): non-immunogenic, + immunogenic

*d* The time required for 50% of the molecules to decay (DT50%) is given starting from t = 2 hours at 37°C. NS = non stable: < 10% of HLA molecules were detectable after a 2 hour incubation at 37°C.

Table v

Comparison of peptide binding affinity, dissociation rate and immunogenicity of HBV and HPV16 derived peptides.

| | Dissociation rate DT50% | |
|---|---|---|
| Peptide binding affinity | ≥ 3 hours | < 3 hours |
| high | 3 | 0 immunogenic |
| | 0 | 0 non-immunogenic |

Table v (continued)

| Comparison of peptide binding affinity, dissociation rate and immunogenicity of HBV and HPV16 derived peptides. | | |
|---|---|---|
| | Dissociation rate DT50% | |
| Peptide binding affinity | ≥ 3 hours | < 3 hours |
| intermediate | 3 | 0 immunogenic |
| | 0 | 4 non-immunogenic |
| low | 0 | 0 immunogenic |
| | 0 | 7 non-immunogenic |

Table VI

The stability of HLA-A*0201 complexed with known CTL epitopes.

| Peptide | First aa position | Sequence | Affinity IC50 (μM) | Stability (DT50%) | | Immunogenicity |
|---|---|---|---|---|---|---|
| | | *a* | *b* | | *c* | *d* |
| HCV1 core | 131 | ADLMGYIPLV | 50.0 | > 4 | hr | RC |
| HCV1 core | 178 | LLALLSCLTV | 7.5 | > 4 | hr | RC |
| HCV1 NS3 | 1406 | KLVALGINAV | 5.0 | 4 | hr | RC |
| HCV1 NS4 | 1789 | SLMAFTAAV | 1.5 | > 4 | hr | RC |
| HBV surface | 335 | WLSLLVPFV | 1.0 | > 4 | hr | RC |
| HBV surface | 348 | GLSPTVWLSV | 2.0 | > 4 | hr | RC |
| EBV LMP2 | 426 | CLGGLLTMV | 2.5 | 4 | hr | PP1 |
| HTLV1 tax | 11 | LLFGYPVYV | 0.8 | > 4 | hr | RC |
| HPV11 E7 | 4 | RLVTLKDIV | 52.0 | 2 | hr | PR2 |
| INF B NP | 85 | KLGEFYNQMM | 5.5 | > 4 | hr | CTL |
| INF A Matrix | 58 | GILGFVFTL | 0.6 | > 4 | hr | CTL |
| HIV-1 Gag | 76 | SLYNTVATL | 1.5 | > 4 | hr | CTL |
| HIV-1 Pol | 267 | VLDVGDAYFSV | 7.0 | NS | | PR3 |
| HIV-1 Pol | 468 | ILKEPVHGV | 8.0 | > 4 | hr | CTL |
| pmel17/gp100 | [1] | YLEPGPVTA | 8.5 | 4 | hr | CTL |
| pmel17/gp100 | [2] | LLDGTATLRL | 5.5 | 4 | hr | CTL |
| tyrosinase | 369 | YMNGTMSQV | 4.5 | > 4 | hr | CTL |

*a* Peptide origin, position of first amino-acid and amino-acid sequence of the different HLA-A*0201 restricted CTL epitopes are given (20,25-36).

*b* Binding affinity was measured as described recently (17). $IC_{50}$ represents the amount of peptide required for 50% inhibition of binding of the fluorescein-labeled reference peptide to HLA-A*0201

*c* The time required for 50% of the molecules to decay (DT50%) is given starting from t = 2 hours at 37°C. NS = non stable, < 10% of HLA molecules were detectable after a 2 hour incubation at 37°C.

*d RC:* recall experiment wherein CTL already primed by viral infection of the patient in vivo were boosted in vitro with peptide to detect the precise epitope. All authors used similar protocols. *CTL:* peptides were used to identify the epitopes recognized by CTL which were obtained from patients. *PR1:* CTL were primed in vitro with an autologous EBV transformed B-cell line and then cloned, peptides were used to map the epitope recognized. *PR2:* CTL were induced in vitro using repeated stimulation with recombinant vaccinia virus-HPV11 E7 infected B-cells. *PR3:* CTL were induced in vitro using repetitive stimulation with peptide pulsed antigen presenting cells.

Table VII

| Statistical analysis of the dissociation rate (DT50%) *or* banding affinity versus immunogenicity of HLA-A*0201 binding peptides. | | | |
|---|---|---|---|
| | immunogenic | non-immunogenic | |
| DT50% ≥ 3 hr | 21 | 0 | |
| DT50% < 3 hr | 2 | 11 | |
| | | | p=0.0000003 [a] |
| high affinity | 11 | 0 | |
| intermediate affinity | 10 | 4 | |
| low affinity | 2 | 7 | |
| | | | p=0.0005 [b] |
| a) Fisher's 2-tailed exact test for 2 by 2 tables. | | | |
| b) The relation between binding affinity and immunogenicity was determined by comparison of the high-affinity binding peptides with the low-affinity binding peptides using a Fisher's 2-tailed axact test for 2 by 2 tables. | | | |
| DT50% ≥ 3 hr | 10 | 0 | |
| DT50% < 3 hr | 2 | 11 | |
| | | | p=0.00007[a] |
| intermediate affinity | 10 | 4 | |
| low affinity | 2 | 7 | |
| | | | p=0.04 [b] |

a)Fisher's 2-tailed exact test for 2 by 2 tables.

Table VIII

| Immunogenicity of HIV-1 derived peptides with known dissociation rate tested in HLA-A*0201/Kb transgenic mice | | | | | | |
|---|---|---|---|---|---|---|
| Sequence+ | origin | Affinity $IC_{50}$ (μM) | Stability DT50% | | LU30%/$10^6$ cells | CTL response |
| | *a* | *b* | | *c* | *d* | *e* |
| FLPSDDFPSV | HBV*core*-18 | 0.4 | > 4 | hr | 53 (25- 71) | 3/3 |
| TLGIVCPI | HPV16*E*7-86 | 0.7 | > 4 | hr | 183 (70-400) | 3/3 |
| VLDVGDAYFSV | HIV-1*pol*-267 | 7.0 | NS | | < 2 | 0/7 |
| YMDDLYVGSDL | HIV-1*pol*-343 | 8.0 | 1 | hr | < 2 | 0/3 |
| LLWKGEGAV | HIV-1*pol*-576 | 6.0 | > 4 | hr | 84 (67-100) | 2/3 |
| ILKEPVHGV | HIV-1*pol*-468 | 8.0 | > 4 | hr | 56 (17-100) | 5/6 |

*a* Peptide amino-acid sequence, protein and position of the first amino-acid of the different HLA-A*0201 binding potential CTL epitopes are given.

*b* Average binding affinity was measured as described recently (17). $IC_{50}$ represents the amount of peptide required for 50% inhibition of binding of the fluorescein-labeled reference peptide to HLA-A*0201

*c* The time required for 50% of the molecules to decay (DT50%) is given starting from t = 2 hours at 37°C. NS = non stable: < 10% of HLA molecules were detectable after a 2 hour incubation at 37°C.

*d* Average of all mice and range of observed responses.

*e* Number of mice which mounted a peptide-specific CTL response per total mice vaccinated.

Table IX:

| **Relation between peptide binding affinity, stability of the MHC peptide complex and immunogenicity of the peptide.** | | | | | |
|---|---|---|---|---|---|
| **Peptide** | **source[1]** | **T2-assay F.I.[2]** | **B cell assay IC50[3]** | **Stability assay DT50% in h.[4]** | **Immuno-genicity[6]** |
| Melan-A/MART-1-derived peptides | | | | | |
| EAAGIGILTV | aa26-35 | 0.58 | 15 | NS[5] | - |
| AAGIGILTV | aa 27-35 | 0.03 | 80 | >4 | + |
| GILTVILGV | aa 31-39 | 0.95 | 6 | >4 | + |
| ALMDKSLHV | as 56-64 | 0.96 | 7 | >4 | + |
| Positive control peptides | | | | | |
| GILGFVFTL | Flu-M1 | 1.35 | 0.6 | >4 | + |
| YLEPGPVTA | pmel17/gp100 | 0.93 | 8.5 | 4 | + |
| LLDGTATLRL | pmel17/gp100 | 0.69 | 4 | 4 | + |
| YMDGTMSQV | tyrosinase | 0.60 | 1.2 | >4 | + |

Legend to Table IX:

1) Protein sources from which peptides have been derived are mentioned. For Melan-A/Mart-1-derived peptides the respective aa positions are indicated. Positive control peptides have been described elswhere (40, 45-47).

2) Fluorescence index (F.I.) is calculated for binding of peptides to T2 cells at 25 µg/ml. The T2 binding assay has been described elswhere (2).

3) Concentration of the peptide that inhibits 50% of maximal binding of reference HBV core peptide. This binding assay employing MHC class I molecules on intact B cells is described under Example 1.

4) Relative stability of peptide binding to HLA-A*0201, calculated as DT50% see Example 2 for experimental procedures).

5) NS = not stable; DT50% not calculated because of absence of peptide binding after 2 hrs.

6) Immunogenicity of peptide (see text Example 3).

Table X

| Preselected peptides having an amino acid sequence derived from human influenza M protein, wherein said amino acid sequence has the ability to bind to human MHC Class I allele HLA-A2.1 and is selected from the group consisting of: | | |
|---|---|---|
| Peptide | Amino acid sequence | location in influenza M protein |
| 1 | S L L T E V E T Y V | (residues 2-11 of M protein) |
| 2 | S L L T E V E T Y V L | (residues 2-12 of M protein) |
| 3 | L L T E V E T Y V | (residues 3-11 of M protein) |
| 4 | L L T E V E T Y V L | (residues 3-12 of M protein) |
| 5 | V L M E W L K T R P I | (residues 41-51 of M protein) |
| 6 | P I L S P L T K G I | (residues 50-59 of M protein) |
| 7 | I L S P L T K G I | (residues 51-59 of M protein) |
| 8 | I L S P L T K G I L | (residues 51-60 of M protein) |
| 9 | G I L G F V F T L | (residues 58-66 of M protein) |
| 10 | G I L G F V F T L T V | (residues 58-68 of M protein) |
| 11 | I L G F V F T L T V | (residues 59-68 of M protein) |
| 12 | R M G A V T T E V | (residues 134-142 of M protein) |
| 13 | G L V C A T C E Q I A | (residues 145-155 of M protein) |
| 14 | Q M V T T T N P L | (residues 164-172 of M protein) |
| 15 | Q M V T T T N P L I | (residues 164-173 of M protein), |

The following table presents preselected peptides derived from human melanoma associated protein tyrosinase capable of upregulating HLA-A*0201-molecules on T2 cells.

Table XI

| Peptide No. | Sequence | Residues |
|---|---|---|
| | CLLWSFQTSA | 008-017 |
| 1 | LLWSFQTSA | 009-017 |
| | RLLVRRNIFDL | 116-126 |
| 2 | YLTLAKHTI | 137-145 |
| | TISSDYVIPI | 144153 |
| | PAFLPWHRLFL | 205-215 |
| 3 | FLPWHRLFL | 207-215 |
| 4 | FLPWHRLFLL | 207-216 |
| 5 | FLLRWEQEI | 214222 |
| 6 | TLEGFASPL | 343-351 |
| 7 | FASPLTGIADA | 347-357 |
| 8 | SMHNALHIYM | 361-370 |
| | HIYMNGTMSQV | 367-377 |
| 9 | YMNGTMSQV | 369-377 |
| | PIFLLHHAFV | 384393 |
| | WLQRHRPLQEV | 400-410 |
| 10 | PLYRNGDFFI | 431-440 |
| 11 | YIKSYLEQA | 463-471 |
| | RIWSWLLGA | 473-481 |
| | RIWSWLLGAAM | 473-483 |
| 12 | WLLGAAMVGA | 477-486 |
| 13 | MVGAVLTAL | 483-491 |
| | VLTALLAGPV | 487-496 |
| | LTALLAGPVSL | 488-498 |
| | TALLAGPVSL | 489-4 |
| | TALLAGPVSLL | 489-499 |
| 14 | ALLAGPVSL | 490-498 |
| 15 | ALLAGPVSLL | 490-499 |
| 16 | LLAGPVSLL | 491-499 |
| | QLPEEKQPLL | 506-515 |
| 17 | LLMEKEDYHSL | 514-524 |

Table XII.

| Preselected peptides having an amino acid sequence derived from p53, wherein said amino -acid sequence has the ability to bind to human MHC Class I allele HLA-A2.1 and is selected from the group consisting of: | | |
|---|---|---|
| Peptide | Amino acid sequence | location in human p53 protein |
| 1. | LLPENNVLS | (residues 25-33 of human p53) |
| 2. | RMPEAAPPV | (residues 65-73 of human p53) |
| 3. | FLHSGTAKSV | (residues 113-122 of human p53) |
| 4. | KMFCQLAKT | (residues 132-140 of human p53) |
| 5. | KQSQHMTEV | (residues 164-172 of human p53) |
| 6. | HMTEVVRRC | (residues 168-176 of human p53) |
| 7. | DRNTFRHSVV | (residues 208-217 of human p53) |
| 8. | LLGRNSFEV | (residues 264-272 of human p53) |
| 9. | KMLCQLAKT | (residues 132-140 of human p53) |
| 10. | NMFCQLAKT | (residues 132-140 of human p53) |
| 11. | KLFCQLAKT | (residues 132-140 of human p53) |

Table XII. (continued)

| Preselected peptides having an amino acid sequence derived from p53, wherein said amino -acid sequence has the ability to bind to human MHC Class I allele HLA-A2.1 and is selected from the group consisting of: | | |
|---|---|---|
| Peptide | Amino acid sequence | location in human p53 protein |
| 12. | QMFCQLAKT | (residues 132-140 of human p53) |
| 13. | KMFTQLAKT | (residues 132-140 of human p53) |
| 14. | KMFYQLAKT | (residues 132-140 of human p53) |
| 15. | KMFCELAKT | (residues 132-140 of human p53) |
| 16. | KMFCQLAKY | (residues 132-140 of human p53) |
| 17. | NLFCQLAKT | (residues 132-140 of human p53) |
| 18. | QQSQHMTEV | (residues 164-172 of human p53) |
| 19. | HMTEVLRRC | (residues 168-176 of human p53) |
| 20. | HMTEVVRLC | (residues 168-176 of human p53) |
| 21. | HMTEVVRRF | (residues 168-176 of human p53) |
| 22. | HMTEVVRHC | (residues 168-176 of human p53) |
| 23. | DRNAFRHSVV | (residues 208-217 of human p53) |
| 24. | DRNTFRHSMV | (residues 208-217 of human p53) |
| 25. | LLVRNSFEV | (residues 264-272 of human p53) |
| 26. | LLGRNSFEM | (residues 264-272 of human p53) |
| A. | ILTIITLED | human p53 residues 251-259 |
| B. | MLSPDDIEQ | human p53 residues 44-52 |
| C. | IRVEGNLRV | human p53 residues 195-203 |
| D. | KLMFKTEGP | human p53 residues 382-390 |
| E. | DLWKLLPEN | human p53 residues 21-29 |
| F. | ALPNNTSSS | human p53 residues 307-315 |
| G. | LHSGTAKSV | human p53 residues 114-122 |
| H. | NLRKKGEPH | human p53 residues 288-297 |
| I. | PLSSSVPSQ | human p53 residues 92-100 |
| J. | ELPPGSTKR | human p53 residues 298-306 |
| K. | FLHSGTAKS | human p53 residues 113-121 |

Preselected peptides having an amino acid sequence derived from HPV16/18 protein, wherein said amino acid sequence has the ability to bind to human MHC Class I allele HLA-A2.1 and is selected from the group consisting of:

Table XIII.

| Peptides derived from HPV16 proteins E6 and E7 binding to HLA-A2.1 | | | |
|---|---|---|---|
| Peptide No. | Amino acid sequence | protein (region) | SEQ NO |
| - | AMFQDPQER | E6 (residues 7- 15) | 1 |
| 1 | KLPQLCTEL | E6 (residues 18 - 26) | 2 |
| 2 | QLCTELQTT | E6 (residues 21 - 29) | 3 |
| 3 | LCTELQTTI | E6 (residues 22 - 30) | 4 |
| 4 | ELQTTIHDI | E6 (residues 25 - 33) | 5 |
| 5 | LQTTIHDII | E6 (residues 26 - 34) | 6 |
| 6 | TIHDIILEC | E6 (residues 29 - 37) | 7 |
| 7 | IHDIILECV | E6 (residues 30 - 38) | 8 |
| 8 | CVYCKQQLL | E6 (residues 37 - 45) | 9 |
| - | FAFRDLCIV | E6 (residues 52- 60) | 10 |
| 9 | KISEYRHYC | E6 (residues 79 - 87) | 11 |
| 10 | PLCDLLIRC | E6 (residues 102-110) | 12 |
| 11 | TLHEYMLDL | E7 (residues 7 - 15) | 13 |
| 12 | YMLDLQPET | E7 (residues 11 - 19) | 14 |

Table XIII. (continued)

| Peptides derived from HPV16 proteins E6 and E7 binding to HLA-A2.1 | | | |
|---|---|---|---|
| Peptide No. | Amino acid sequence | protein (region) | SEQ NO |
| 13 | MLDLQPETT | E7 (residues 12 - 20) | 15 |
| 14 | RLCVQSTHV | E7 (residues 66 - 74) | 16 |
| 15 | TLEDLLMGT | E7 (residues 78 - 86) | 17 |
| 16 | LLMGTLGIV | E7 (residues 82 - 90) | 18 |
| 17 | GTLGIVCPI | E7 (residues 85 - 93) | 19 |
| 18 | TLGIVCPIC | E7 (residues 86 - 94) | 20 |

TABLE XIV

| Peptides derived from HPV18 proteins E6 and E7 binding to HLA-A2.1 | | | |
|---|---|---|---|
| Peptide No. | Amino acid sequence | protein (region) | SEQ NO |
| 1 | KLPDLCTEL | E6 (residues 13 - 21) | 21 |
| 2 | SLQDIEITC | E6 (residues 24 - 32) | 22 |
| 3 | LQDIEITCV | E6 (residues 25 - 33) | 23 |
| 4 | EITCVYCKT | E6 (residues 29 - 37) | 24 |
| 5 | KTVLELTEV | E6 (residues 36 - 44) | 25 |
| 6 | ELTEVFEFA | E6 (residues 40 - 48) | 26 |
| 7 | FAFKDLFVV | E6 (residues 47 - 55) | 27 |
| 8 | DTLEKLTNT | E6 (residues 88 - 96) | 28 |
| 9 | LTNTGLYNL | E6 (residues 93 -101) | 29 |
| 10 | TLQDIVLHL | E7 (residues 7 - 15) | 30 |
| 11 | FQQLFLNTL | E7 (residues 86 - 94) | 31 |
| 12 | QLFLNTLSF | E7 (residues 88 - 96) | 32 |
| 13 | LFLNTLSFV | E7 (residues 89 - 97) | 33 |
| 14 | LSFVCPWCA | E7 (residues 94 -102) | 34 |

TABLE XV

| Peptides derived from HPV16 proteins E6 and E7 binding to HLA-A1 | | |
|---|---|---|
| Amino acid sequence | protein (region) | SEQ NO |
| YRDGNPYAV | E6 (residues 61- 69) | 35 |
| WTGRCMSCC | E6 (residues 139-147) | 36 |
| MSCCRSSRT | E6 (residues 144-152) | 37 |
| TTDLYCYEQ | E7 (residues 19- 27) | 38 |
| EIDGPAGQA | E7 (residues 37- 45) | 39 |
| HVDIRTLED | E7 (residues 73- 81) | 40 |

TABLE XVI

| Peptides derived from HPV16 proteins E6 and E7 binding to HLA-A3.2 | | |
|---|---|---|
| Amino acid sequence | protein (region) | SEQ NO |
| AMFQDPQER | E6 (residues 7- 15) | 1 |
| IILECVYCK | E6 (residues 33- 41) | 41 |
| CVYCKQQLL | E6 (residues 37- 45) | 9 |
| VYCKQQLLR | E6 (residues 38- 46) | 42 |
| QQLLRREVY | E6 (residues 42- 50) | 43 |

TABLE XVI (continued)

| Peptides derived from HPV16 proteins E6 and E7 binding to HLA-A3.2 | | |
|---|---|---|
| Amino acid sequence | protein (region) | SEQ NO |
| IVYRDGNPY | E6 (residues 59- 67) | 44 |
| YAVCDKCLK | E6 (residues 67- 75) | 45 |
| AVCDKCLKF | E6 (residues 68- 76) | 46 |
| VCDKCLKFY | E6 (residues 69- 77) | 47 |
| KFYSKISEY | E6 (residues 75- 83) | 48 |
| KISEYRHYC | E6 (residues 79- 87) | 11 |
| ISEYRHYCY | E6 (residues 80- 88) | 49 |
| RHYCYSLYG | E6 (residues 84- 92) | 50 |
| SLYGTTLEQ | E6 (residues 89- 97) | 51 |
| TTLEQQYNK | E6 (residues 93-101) | 52 |
| QQYNKPLCD | E6 (residues 97-105) | 53 |
| LIRCINCQK | E6 (residues 107-115) | 54 |
| HLDKKQRFH | E6 (residues 125-133) | 55 |
| CMSCCRSSR | E6 (residues 143-151) | 56 |
| SCCRSSRTR | E6 (residues 145-153) | 57 |
| CCRSSRTRR | E6 (residues 146-154) | 58 |
| HYNIVTFCC | E7 (residues 51- 59) | 59 |
| YNIVTFCCK | E7 (residues 52- 60) | 60 |
| CCKCDSTLR | E7 (residues 58- 66) | 61 |
| KCDSTLRLC | E7 (residues 60- 68) | 62 |

TABLE XVII

| Peptides derived from HPV16 proteins E6 and E7 binding to HLA-A11.2 | | |
|---|---|---|
| Amino acid sequence | protein (region) | SEQ NO |
| AMFQDPQER | E6 (residues 7- 15) | 1 |
| IILECVYCK | E6 (residues 33- 41) | 41 |
| CVYCKQQLL | E6 (residues 37- 45) | 9 |
| VYCKQQLLR | E6 (residues 38- 46) | 42 |
| QQLLRREVY | E6 (residues 42- 50) | 43 |
| IVYRDGNPY | E6 (residues 59- 67) | 44 |
| YAVCDKCLK | E6 (residues 67- 75) | 45 |
| AVCDKCLKF | E6 (residues 68- 76) | 46 |
| VCDKCLKFY | E6 (residues 69- 77) | 47 |
| KISEYRHYC | E6 (residues 79- 87) | 11 |
| ISEYRHYCY | E6 (residues 80- 88) | 49 |
| LIRCINCQK | E6 (residues 107-115) | 54 |
| TGRCMSCCR | E6 (residues 140-148) | 63 |
| CMSCCRSSR | E6 (residues 143-151) | 56 |
| SCCRSSRTR | E6 (residues 145-153) | 57 |
| HYNIVTFCC | E7 (residues 51- 59) | 59 |
| YNIVTFCCK | E7 (residues 52- 60) | 60 |
| CCKCDSTLR | E7 (residues 58- 66) | 61 |
| VCPICSQKP | E7 (residues 90- 98) | 64 |

TABLE XVIII

| Peptides derived from HPV16 proteins E6 and E7 binding to HLA-A24 | | |
|---|---|---|
| Amino acid sequence | protein (region) | SEQ NO |
| MHQKRTAMF | E6 (residues 1- 9) | 65 |
| LQTTIHDII | E6 (residues 26- 34) | 6 |
| VYCKQQLLR | E6 (residues 38- 46) | 42 |
| LLRREVYDF | E6 (residues 44- 52) | 66 |
| VYDFAFRDL | E6 (residues 49- 57) | 67 |
| PYAVCDKCL | E6 (residues 66- 74) | 68 |
| KCLKFYSKI | E6 (residues 72- 80) | 69 |
| EYRHYCYSL | E6 (residues 82- 90) | 70 |
| HYCYSLYGT | E6 (residues 85- 93) | 71 |
| CYSLYGTTL | E6 (residues 87- 95) | 72 |
| RFHNIRGRW | E6 (residues 131-139) | 73 |
| RAHYNIVTF | E7 (residues 49- 57) | 74 |

Table XIX

| Preselected peptides having an amino acid sequence derived from HIV, wherein said amino acid sequence has the ability to bind to human MHC Class I allele HLA-A2.1 and is selected from the group consisting of: | |
|---|---|
| 1. | E M M T A C Q G V |
| 2. | L L D T G A D D T V |
| 3. | V L D V G D A Y F S V |
| 4. | L L W K G E G A V |
| 5. | I L K E P V H G V |

Table XX.

| Preselected peptides having an amino acid sequence derived from CEA, wherein said amino-acid sequence has the ability to bind to human MHC Class I allele HLA-A2.1 and is selected from the group consisting of: | | |
|---|---|---|
| Peptide no. | Amino acid sequence | Residues |
| A1 | Q I I G Y V I G T | 044-052 |
| A2 | Y L W W V N N Q S L | 142-151 |
| A3 | V L Y G P D A P T I | 199-208 |
| A4 | V L Y G P D T P I | 555-563 |
| A5 | Y L S G A N L N L | 571-579 |
| Peptides derived from CEA binding to the HLA-A*0301 molecule | | |
| Peptide no. | Amino acid sequence | Residues |
| B1 | H L F G Y S W Y K | 027-035 |
| B2 | R V D G N R Q I I G Y | 038-048 |
| B3 | R V Y P E L P K | 105-112 |
| B4 | R L Q L S N D N R | 334-342 |
| B5 | E L F I S N I T E K | 427-436 |
| B6 | L F I S N I T E K | 428-436 |
| B7 | F I S N I T E K | 429-436 |
| B8 | T L T L F N V T R | 521-529 |
| B9 | T L F N V T R N D A R | 523-533 |
| B10 | N V T R N D A R | 526-533 |
| B11 | F V S N L A T G R | 622-630 |

Table A the oligonucleoties used in these studies in order to generate rAdV that encoded CTL epitopes in a string-of-bead fashion, linked with proteolytic cleavage sites that direct CTL epitope processing.

| | |
|---|---|
| 1a | CGCGAATTATGAACGCGTC |
| 1b | GTACGACGCGTRCATAATT |
| 2a | GTACGCTACTAGTGAACAGAAGCTGATATCAGAGGAAGACCTAAACTGAT |
| 2b | CTAGATCAGTTTAGG CWMATATCAGCTTCTGTTCACTAGTAGC |
| 3a | CGCGGCAGCTTCCGGTCCTTCTAACACACCTCCTGAGATAGCAGCC |
| 3b | GCTATCTCAGGAGGTGTGTTAGAAGGACCGGAAGCTGC |
| 4a | CTGTAAATATCAGGAATTGTTGCTACATTGCAGCTG |
| 4b | CTAGCAGCTGCAATGTAGCAACAATTCCTGATATTTACAGCTGC |
| 5a | CGCGGCAGCTACACTAGGAATTGTGTGCCCCATCGCAGCC |
| 5b | GCGATGGGGCACACAATTCCTAGTGTAGCTGC |
| 6a | GCTAGAGCCCATTACAATATTGTAACCTTTGCTGCG |
| 6b | GCAAAGGTTACAATATTGTAATGGGCTCTAGCGGCT |
| 7a | GCTGCCATCTACAAGAAGTCACAGCACATGGCTGCAG |
| 7b | ACGCCGACGGTAGATGTTCTTCAGTGTCGTGTACCGA |
| 8a | GCTGGAATCCTAGGTTTCGTCTTTACGCTAGCTGCG |
| 8b | GCTAGAGTAAAGACGAAACCTAGGATTCCAGCGGCT |
| 9a | GCTTATATGTTAGATTTGCAACCAGAGACAACTGCTGCAG |
| 9b | AGCAGTTGTCTCTGGTTGCAAATCTAACATATAAGCCGCA |

**Claims**

**1.** A method for the selection of immunogenic T cell peptide epitopes present in polypeptide antigens comprising identification of peptides in the primary sequence of the antigen having a binding motif and size for binding to a HLA class I molecule, measuring the binding of said identified peptides to MHC class I molecules, whereby the stability of the complex of the peptide and the MHC class I molecule is measured on intact cells carrying said MHC class I molecule at their surfaces.

**2.** A method according to claim 1 whereby the intact cells are B cells.

**3.** A method according to claim 2 whereby the B cells are human B cells.

**4.** A method according to any one of the aforegoing claims comprising a further selection step of peptides whereby the peptides and their flanking sequences in the intact antigen are screened for compliance with the rules for proteasome cleavage of natural polypeptides sequences.

**5.** A method according to any one of the aforegoing claims whereby the peptides and their flanking sequences in the intact antigen are screened for compliance with the rules for peptide transport and/or peptide loading onto HLA class I molecules.

**6.** A method according to any one of the aforegoing claims further comprising a further binding assay for the binding of identified peptides to MHC class I molecules.

**7.** A method according to claim 6 wherein the further binding assay measures the binding of identified peptides to empty MHC class I molecules at the surface of an antigen processing defective cell line.

**8.** A method according to claim 7 wherein the processing defective cell line is the T2 cell line.

**9.** A method for the production of a vaccine for the treatment and/or prophylaxis of a disease associated with the presence of a polypeptide antigen, comprising selecting T cell peptide epitopes of said polypeptide antigen by a method according to any one of claims 1-8, preparing selected peptide epitopes and mixing said peptide epitopes with a vehicle suitable for administration.

**10.** A method for producing a vaccine according to claim 9 whereby an adjuvans is added to the vaccine.

**11.** A method according for producing a vaccne according to claim 9 or 10, whereby the peptide epitope comprises a synthetic peptide.

**12.** A method according to claim 11 whereby the vaccine comprises a mixture of different synthetic peptides.

**13.** A method according to claim 11 or 12 whereby the synthetic peptide is loaded onto a dendritic cell.

**14.** A method according to anyone of laims 9-12, whereby the peptide epitopes are present in a string-bead conformation.

**15.** A method according to claim 14 whereby the strings comprises proteolytic cleavage sites.

**16.** A method according to claim 9 or 10 whereby a peptide epitope is provided as part of a recombinant protein.

**17.** A method according to claim 16 whereby the recombinant protein has a string bead conformation whereby the beads are peptide epitopes.

**18.** A method according to claim 17 whereby the strings comprise proteolytic cleavage sites.

**19.** A method according to any one of claims 16-18 whereby the recombinant protein is loaded on a dendritic cell.

**Patentansprüche**

**1.** Verfahren zum Auswählen von immunogenen T-Zell-Peptid-Epitopen, die in Polypeptidantigenen vorhanden sind, umfassend die Identifikation von Peptiden in der Primärsequenz des Antigens, die ein Bindungsmotiv und die Größe für die Bindung an ein HLA-Klasse-I-Molekül haben, das Messen der Bindung der identifizierten Peptide an MHC-Klasse-I-Moleküle, wobei die Stabilität des Komplexes aus dem Peptid und dem MHC-Klasse-I-Molekül an intakten Zellen gemessen wird, die das MHC-Klasse-I-Molekül auf ihrer Oberfläche tragen.

2. Verfahren gemäß Anspruch 1, wobei die intakten Zellen B-Zellen sind.

3. Verfahren gemäß Anspruch 2, wobei die B-Zellen humane B-Zellen sind.

4. Verfahren gemäß einem der vorigen Ansprüche, das einen weiteren Selektionsschritt von Peptiden umfasst, wobei die Peptide und ihre flankierenden Sequenzen im intakten Antigen daraufhin durchmustert werden, ob sie den Regeln für die Proteasomspaltung natürlicher Polypeptidsequenzen entsprechen.

5. Verfahren gemäß einem der vorigen Ansprüche, wobei die Peptide und ihre flankierenden Sequenzen in dem intakten Antigen daraufhin durchmustert werden, ob sie den Regeln für Peptidtransport und/oder Peptidbeladung auf HLA-Klasse-I-Molekülen entsprechen.

6. Verfahren gemäß einem der vorigen Ansprüche, das weiterhin einen weiteren Bindungsassay für die Bindung von identifizierten Peptiden an MHC-Klasse-I-Moleküle umfasst.

7. Verfahren gemäß Anspruch 6, wobei der weitere Bindungsassay die Bindung identifizierter Peptide an leere MHC-Klasse-I-Moleküle an der Oberfläche einer antigenprozessierungsdefekten Zelllinie misst.

8. Verfahren gemäß Anspruch 7, wobei es sich bei der prozessierungsdefekten Zelllinie um die T2-Zelllinie handelt.

9. Verfahren zur Herstellung eines Impfstoffs zur Behandlung und/oder Prophylaxe einer Krankheit, die mit der Anwesenheit eines Polypeptidantigens verbunden ist, umfassend das Auswählen von T-Zell-Peptid-Epitopen des Polypeptidantigens durch ein Verfahren gemäß einem der Ansprüche 1-8, das Präparieren ausgewählter Peptidepitope und das Mischen der Peptidepitope mit einem für die Verabreichung geeigneten Träger.

10. Verfahren zur Herstellung eines Impfstoffs gemäß Anspruch 9, wobei ein Adjuvans zu dem Impfstoff gegeben wird.

11. Verfahren zur Herstellung eines Impfstoffs gemäß Anspruch 9 oder 10, wobei das Peptidepitop ein synthetisches Peptid umfasst.

12. Verfahren gemäß Anspruch 11, wobei der Impfstoff ein Gemisch von verschiedenen synthetischen Peptiden umfasst.

13. Verfahren gemäß Anspruch 11 oder 12, wobei das synthetische Peptid auf eine dendritische Zelle geladen wird.

14. Verfahren gemäß einem der Ansprüche 9-12, wobei die Peptidepitope in einer Perlenkettenkonformation vorliegen.

15. Verfahren gemäß Anspruch 14, wobei die Ketten proteolytische Spaltungsstellen umfassen.

16. Verfahren gemäß Anspruch 9 oder 10, wobei ein Peptidepitop als Teil eines rekombinanten Proteins bereitgestellt wird.

17. Verfahren gemäß Anspruch 16, wobei das rekombinante Protein eine Perlenkettenkonformation hat, wobei die Perlen Peptidepitope sind.

18. Verfahren gemäß Anspruch 17, wobei die Ketten proteolytische Spaltungsstellen umfassen,

19. Verfahren gemäß einem der Ansprüche 16-18, wobei das rekombinante Protein auf eine dendritische Zelle geladen ist.

## Revendications

1. Un procédé pour la sélection d'épitopes peptidiques immunogènes de cellules T présents dans des antigènes polypeptidiques, comprenant l'identification de peptides dans la séquence primaire de l'antigène ayant un motif de liaison et une taille convenant pour la liaison à une molécule HLA de classe I, la mesure de la liaison desdits peptides identifiés à des molécules de la classe I du CMH, dans lequel la stabilité du complexe du peptide et de la molécule de la classe I du CMH est mesurée sur des cellules entières portant ladite molécule de la classe I du CMH à leurs surfaces.

**2.** Un procédé selon la revendication 1, dans lequel les cellules entières sont des cellules B.

**3.** Un procédé selon la revendication 2, dans lequel les cellules B sont des cellules B humaines.

**4.** Un procédé selon l'une quelconque des revendications précédentes, comprenant une autre étape de sélection de peptides dans laquelle les peptides et leurs séquences adjacentes dans l'antigène entier sont criblés pour la conformité aux règles de clivage par procéasome des séquences polypeptidiques naturelles.

**5.** Un procédé selon l'une quelconque des revendications précédentes, dans lequel les peptides et leurs séquences adjacentes dans l'antigène entier sont criblés pour la conformité aux règles de transport de peptide et/ou de mise en place de peptide sur les molécules HLA de la classe I.

**6.** Un procédé selon l'une quelconque des revendications précédentes, comprenant de plus un autre essai de liaison pour déterminer la liaison des peptides identifiés à des molécules de la classe I du CMH.

**7.** Un procédé selon la revendication 6, dans lequel l'autre essai de liaison mesure la liaison des peptides identifiés à des molécules vides de la classe I du CMH à la surface d'une lignée cellulaire déficiente pour le traitement de l'antigène.

**8.** Un procédé selon la revendication 7, dans lequel la lignée cellulaire déficience pour le traitement est la lignée cellulaire T2.

**9.** Un procédé pour la production d'un vaccin destiné au traitement et/ou à la prophylaxie d'une maladie associée à la présence d'un antigène polypeptidique, comprenant la sélection d'épitopes peptidiques de cellules T dudit antigène polypeptidique par un procédé selon l'une quelconque des revendications 1 à 8, la préparation des épitopes peptidiques sélectionnés et le mélange desdits épitopes peptidiques avec un véhicule approprié pour l'administration.

**10.** Un procédé pour la production d'un vaccin selon la revendication 9, dans lequel un adjuvant est ajouté au vaccin.

**11.** Un procédé pour la production d'un vaccin selon la revendication 9 ou 10, dans lequel l'épitope peptidique comprend un peptide synthétique.

**12.** Un procédé selon la revendication 11, dans lequel le vaccin comprend un mélange de différents peptides synthétiques.

**13.** Un procédé selon la revendication 11 ou 12, dans lequel le peptide synthétique est mis en place sur une cellule dendritique.

**14.** Un procédé selon l'une quelconque des revendications 9 à 12, dans lequel les épitopes peptidiques sont présents dans une conformation en perles et cordons.

**15.** Un procédé selon la revendication 14, dans lequel les cordons comprennent des sites de clivage protéolytique.

**16.** , Un procédé selon la revendication 9 ou 10, dans lequel un épitope peptidique est fourni en tant que partie d'une protéine recombinante.

**17.** Un procédé selon la revendication 16, dans lequel la protéine recombinante a une conformation en perles et cordons dans laquelle les perles sont des épitopes peptidiques.

**18.** Un procédé selon la revendication 17, dans lequel les cordons comprennent des sites de clivage protéolytique.

**19.** Un procédé selon l'une quelconque des revendications 16 à 18, dans lequel la protéine recombinante est mise en place sur une cellule dendritique.

% relative binding
225
200
175
150
125
100
75
50
25
0
KR (A3 . B7 . DR7. DQw2 )
B109 (A1 .A23.B7 .B45 .Cw6.Cw7 .DR4 .DRw53.DQw7.DQw8)
D110 (A1 .A23.B8 .B44 .Cw4.Cw7 .DR3 .DR7 .DQw2 )
JY (A2 . B7 . Cw7. DR4 .DRw6 .DPw2 )
D100 (A11.A24.B7 .Bw62.Cw7.Cw9 .DR4 .DRw6 .DQw6.DQw7)
B109 (A1 .A23.B7 .B45 .Cw6.Cw7 .DR4 .DRw53.DQw7.DQw8)
D100 (A11.A24.B7 .Bw62.Cw7.Cw9 .DR4 .DRw6 .DQw6.DQw7)
D110 (A1 .A23.B8 .B44 .Cw4.Cw7 .DR3 .DR7 .DQw2 )
S99 (A24.A32.B7 .B14 . DRw10.DRw15.DQw5.DQw6)
S59 (A2 .A26.B18.B51 .Cw1.Cw9 .DRw11.DRw16.DQw2.DQw6)
K97 (A3 .A32.B7 .B14 .Cw7. DR7 . .DQw2.DQw6)
P98 (A3 .A24.B7 .B37 .Cw6.Cw7 .DRw13.DRw15.DQw6 )
BRM (A2 .A11.B35.B62 .Cw4.Cw10.DR4 .DR7 .DQw2.DQw7)
ML (A2 .A11.B27.B62 .Cw2.Cw3 .DR7 .DR14 .DQw1.DQw2)
NL (A2 .A28.B7 .B35 .Cw4.Cw7 .DR1 . DQw5 )

FIG. 1

MF
100
80
60
40
20
0
0
50
100
150
200
250
300
350
400
450
500
nM

FIG. 2

FI
4
3
2
1
0
0
60
120
180
240
300
360
Time (minutes)

FIG. 3

Fluorescence index
10
8
6
4
2
0
26°C
4°C
1
3
4,5
1
3
4,5
incubation in hours

FIG. 4

kvfpC(FL)alink 150 nM
flpsdC(FL)fpsv 150 nM
% inhibition
competitor peptide μM
competitor peptide μM
0
20
40
60
80
100
0
5
10
15
20
25
30
35
40
45
50

FIG. 5

relative dissociation
0
1
2
3
0
2
4
6
8
time (hours)

% inhibition
0
20
40
60
80
100
0
20
40
60
80
100
competitor peptide μM

FIG. 6

A
° FLPSDDFPSV
• No peptide
B
° ILKEPVHGV
• No peptide
C
° VLDVGDAYFSV
• No peptide
D
° YMDDLYVGSDL
• No peptide
E
° LLWKGEGAV
• No peptide
% specific $^{51}$Cr-release
E:T ratio
0
10
20
30
40
50
60
70
80
100

FIG. 7

Recombinant Adenoviruses carrying 'string bead' minigenes
rAd-1
Ad MLP
MNAAASGPSNTPPEIAAARAHYNIVTFAAAIYKKSQHMAAAVNIRNCCYIAAASEQKLISEEDLNN
Ad5E1A
H-2Db
HPV16 E7
H-2Db
wt-p53
H-2Kb
Ad5E1B
H-2Db
hu-c-Myc
MAb 9E10
rAd-2
Ad MLP
MNAAATLGIVCPIAAAGILGFVFTLAAAYMDLQPETTAAAVNIRNCCYIAAASEQKLISEEDLNN
HPV16 E7
HLA-A*0201
Flu-matrix
HLA-A*0201
HPV16 E7
HLA-A*0201
Ad5E1B
H-2Db
hu-c-Myc
MAb 9E10

Figure 8

CTL epitopes encoded by string-bead minigene 1
are processed and presented to tumor-specific CTL
Ad5E1A-specific CTL
HPV16 E7-specific CTL
p53-specific CTL
Ad5E1B-specific CTL
% Specific WEHI-cell death
100
75
50
25
0
Db
Ad5E1 + Db
minigene 1 + Db
minigene 2 + Db
HPV16 E7 + Db
Kb
p53 + Kb
minigene 1 + Kb
minigene 2 + Kb

CTL epitopes encoded by string-bead minigene 2 are processed and presented to tumor-specific CTL

Flu-specific CTL

Ad5E1B-specific CTL

% Specific WEHI-cell death

100
75
50
25
0

A2
Flu + A2
minigene 1 + A2
minigene 2 + A2

Db
Ad5E1 + Db
minigene 1 + Db
minigene 2 + Db

Figure 10

CTL epitopes encoded by rAd in a string-bead fashion are processed and presented to tumor-specific CTL
% Specific WEHI-cell death
100
80
60
40
20
0
---
rAd-1
rAd-2
rAd-LacZ
Effectors:
---
Ad5E1A-specific CTL
HPV16 E7-specific CTL
Ad5E1B-specific CTL

Figure 11

Ad5E1-specific CTL immunity induced by vaccination with rAd-1 or rAd-2

In vivo: non-immunized
In vitro: Ad5E1 MEC

MEC + Sendai-peptide
MEC + Ad5E1A-peptide
MEC + Ad5E1B-peptide
MEC + HPV16 E7-peptide
Ad5E1 MEC

% Specific lysis

E/T

In vivo: rAd-1
In vitro: Ad5E1 MEC

E/T

In vivo: rAd-2
In vitro: Ad5E1 MEC

E/T

Figure 12

HPV16 E7-specific, H-2b-restricted CTL immunity induced by vaccination with rAd-1
In vivo: non-immunized
In vitro: HPV16 MEC
MEC + Sendai-peptide
MEC + Ad5E1A-peptide
MEC + Ad5E1B-peptide
MEC + HPV16 E7-peptide
HPV16 MEC
% Specific lysis
60
45
30
15
0
0
15
30
45
60
E/T
In vivo: rAd-1
In vitro: HPV16 MEC
60
40
20
0
0
15
30
45
60
E/T
In vivo: rAd-2
In vitro: HPV16 MEC
60
40
20
0
0
15
30
45
60
E/T

Figure 13

Vaccination with rAd-1 induces protective immunity against Ad5E1A + ras tumors
% Survival
100
75
50
25
0
15
25
35
45
55
Days
Ad5E1A + ras MEC
PBS/BSA
rAd-1
rAd-2
Ad5 ts149

Figure 14